# FASCICULE DE BREVET EUROPEEN

(11) **EP 1 475 376 B1**
(45) Date de publication et mention de la délivrance du brevet: **18.07.2007**
(21) Numéro de dépôt: 03292979.6
(22) Date de dépôt: 28.11.2003
(51) Int. Cl.: C07D 401/04

(54) **Composition capillaire contenant un dérivé de pyrimidine N-oxyde, son utilisation pour stimuler la pousse des fibres kératiniques et/ou freiner leur chute**
Haarpflegemittel enthaltend ein Pyrimidin-N-Oxid-Derivate und Verwendung zur Stimulation von keratinischem Faserwachstum und/oder zur Verhinderung von dessen Verlust
Hair composition containing a pyrimidine-n-oxide derivative and its use to stimulate keratinic fiberes growth and/or preventing their loss

(30) Priorité: 06.05.2003 FR 0350145
(43) Date de publication de la demande: 10.11.2004
(73) Titulaire: L'ORÉAL, 75008 Paris (FR)
(72) Inventeur: Dalko, Maria, 91190 Gif S/Yvette (FR); Mahe, Yann, 91390 Morsang sur Orge (FR); Cals-Grierson, Marie-Madeleine, 92190 Meudon (FR)
(74) Mandataire: Wattremez, Catherine

(56) Documents cités:
- EP-A- 0 408 442
- EP-A- 0 459 890
- EP-A- 0 522 964
- US-A- 5 328 914

## Description

### DOMAINE DE L'INVENTION

L'invention a pour objet une composition cosmétique ou pharmaceutique contenant une quantité efficace d'un composé pyrimidine N-oxyde, destinée à induire et/ou stimuler la croissance des fibres kératiniques humaines et en particulier des cheveux et les cils et/ou freiner leur chute. Elle se rapporte, en outre, à un procédé de traitement cosmétique destiné à stimuler la croissance des fibres kératiniques humaines telles que les cheveux et les cils et/ou freiner leur chute et à l'utilisation de ce composé pyrimidine N-oxyde comme actif donneur de NO et/ou inhibiteur de lysyl-hydroxylase, destiné en particulier à induire et/ou stimuler la croissance des cheveux ou des cils et/ou freiner leur chute.

L'invention a aussi pour objet de nouveaux dérivés de pyrimidine N-oxyde, présentant une activité donneur de NO et/ou inhibiteur de lysyl-hydroxylase.

### ARRIERE PLAN DE L'INVENTION

La croissance des cheveux et leur renouvellement sont principalement déterminés par l'activité des follicules pileux et de leur environnement matriciel. Leur activité est cyclique et comporte essentiellement trois phases à savoir la phase anagène, la phase catagène et la phase télogène.

A la phase anagène (phase active ou de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10% environ sont au repos et seront remplacés en quelques mois.

A l'âge adulte, le système vasculaire de la peau est parachevé et ne se modifie plus sauf au niveau des follicules pileux où il subit d'importantes variations à chaque cycle pilaire. Les follicules pileux sont en effet une structure cutanée richement innervée et très vascularisée. Le phénomène de développement de la microcirculation des follicules pileux est appelé angiogénèse. Au début de chaque phase anagène, il est nécessaire de développer une forte activation de l'angiogénèse afin de re-développer le microréseau vasculaire périfolliculaire. L'involution de ce microréseau et la disparition des capillaires sanguins de la papille dermique vont de pair avec le changement de phase et le passage en phase catagène. A ce stade, les microvaisseaux sanguins collapsent et disparaissent.

Parallèlement, dans les zones alopéciques, une fibrose périfolliculaire s'installe, les follicules se miniaturisent cycle après cycle et progressivement, la vascularisation spécifique des bulbes s'amoindrie.

Le phénomène d'angiogénèse observé au cours de la phase anagène dépend de nombreux facteurs trophiques, de cytokines ou d'autres molécules biologiquement actives apportées par la circulation sanguine ou localement produites en particulier par les fibroblastes de la papille dermique ou les kératinocytes du bulbe capillaire. Parmi ces facteurs trophiques on peut citer le facteur de croissance des cellules endothéliales, (appelé également vascular endothelial growth factor (VEGF) en terminologie anglo-saxonne). Ce facteur est essentiel pour l'angiogénèse et augmente la perméabilité vasculaire. Des études ont montré que l'expression de ce facteur était augmentée au cours de la phase anagène du cycle pilaire. Ainsi, ce facteur contribue au maintien d'une microvascularisation fonctionnelle autour du follicule pileux et notamment de la base du bulbe et de la papille dermique, ainsi qu'à l'apport de nutriments nécessaire à la bonne croissance du cheveu.

La microcirculation périfolliculaire joue donc un rôle primordial dans le processus de croissance pilaire en apportant les facteurs et les nutriments nécessaires à la croissance de ce follicule.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

Par ailleurs, dans certaines dermatoses du cuir chevelu à caractéristique inflammatoire, telles que par exemple le psoriasis ou les dermatites séborrhéiques, la chute des cheveux peut être fortement accentuée et les cycles de renouvellement des follicules peuvent être fortement perturbés.

En outre, différentes causes peuvent entraîner une perte importante, temporaire ou définitive, des cheveux. Il peut s'agir de chute et d'altération des cheveux au décours d'une grossesse (post partum), au cours d'états de dénutrition ou de déséquilibres alimentaires ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Il se produit alors un appauvrissement progressif de la chevelure et une miniaturisation progressive des cheveux, conjointement à un isolement des bulbes par une progression de l'épaisseur de la matrice collagénique périfolliculaire et de la gaine conjonctive externe. La revascularisation est donc rendue plus difficile cycle après cycle. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme et, chez la femme, on constate une alopécie diffuse du vertex.

De par le rôle primordial de la microcirculation périfolliculaire énoncé plus haut, tout défaut de cette dernière entraînera une diminution de l'apport des éléments nutritifs et gazeux (Oxygène notamment) nécessaires à la croissance pilaire conduisant à des troubles de la croissance du cheveu et la mise en place progressive d'une alopécie.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique ou la malnutrition peuvent accentuer le phénomène de chute des cheveux.

De façon générale, tout facteur entraînant une augmentation de l'apport sanguin au niveau du follicule pileux soit en activant l'angiogénèse, soit en s'opposant à sa régression, soit encore en agissant sur les microvaisseaux pour limiter leur constriction, aura un effet bénéfique sur l'apport énergétique nécessaire à la bonne croissance de ce même follicule.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute. L'une des voies explorées est le maintien de la vascularisation autour du follicule pileux.

Ainsi, un des composés connu pour maintenir la vascularisation périfolliculaire est le vérapamil, qui est un antagoniste puissant (IC_{50Ca2+} = 38nM) des canaux calciques de type L. Le vérapamil et d'autres antagonistes de canaux calciques comme le diltiazem et la nifédipine sont décrits comme étant actifs dans le traitement de la chute des cheveux, en particulier par leurs effets sur la microcirculation (confère les documents de Shiseido JP88/062680 et de Coppe J. BE/89/000305). IC_{50Ca2+} représente la concentration inhibitrice de 50 % de libération de Ca²⁺.

En outre, il existe des documents décrivant l'utilisation de donneurs de NO (monoxyde d'azote) pour application sur le cuir chevelu, pour stimuler la croissance de cheveux en agissant sur la microcirculation du cuir chevelu. Ainsi, le document brevet de Proctor (EP 0327263) décrit l'utilisation de composés producteurs du radical NO, en association avec des agents réducteurs, des anti-oxydants et avec des piégeurs de radical hydroxyle. Un autre document brevet de E. Fossel (WO 99/13717) décrit l'utilisation de l'arginine et de ses dérivés, comme substrat de NO-synthase, pour la formation *in vivo* de NO et leur utilisation (entre autre) dans le traitement de l'alopécie. Un autre document brevet de Shiseido (JP-A-07316023) décrit également l'utilisation de l'arginine et de ses dérivés dans le traitement de l'alopécie.

Ces substances connues présentent des effets indésirables. En particulier, elles présentent des activités multiples, pouvant perturber l'équilibre ionique et physiologique des cellules cutanées. Autrement dit, leur activité multiple rend difficile leur contrôle d'action sur les cellules.

Le présente invention se rapporte à l'utilisation de nouveaux composés particuliers permettant de remédier aux inconvénients ci-dessus. Ces composés présentent en outre, une activité locale, spécifique. Ils assurent une revascularisation du follicule pileux, de sa région périphérique et/ou du cheveu après chaque cycle de croissance, ainsi qu'une inhibition de la formation et de l'installation de la fibrose périfolliculaire, facteur aggravant une mauvaise vascularisation du cheveu.

Comme molécule connue agissant spécifiquement sur la formation et l'installation de la fibrose périfolliculaire, on peut citer l'aminexil (2-4 diamino pyrimidine N-oxyde), décrite dans le document WO 96/09048 et qui a une activité inhibitrice de l'expression de la lysyl-hydroxylase. La lysyl-hydroxylase et la prolyl-hydroxylase sont des enzymes impliqués dans la formation des fibres de collagènes, le dérèglement de leurs fonctionnements est un des facteurs responsable du développement de la fibrose périfolliculaire.

On connaît aussi du document GB-A-2198132 l'utilisation des dérivés 2,6 diamino pyrimidine N-oxyde dans des compositions induisant et stimulant la croissance des cheveux et réduisant la chute des cheveux. On connaît également des dérivés de pyrimidine N-oxyde dans les documents EP-A-0408 442, EP-A-0459890, US-A-5328914 et EP-A-0522964 dans des compositions induisant et stimulant la croissance des cheveux et réduisant la chute des cheveux.

Ainsi, après de nombreux efforts, le demandeur a mis au point de nouveaux dérivés pyrimidine N-oxyde possédant des activités locales spécifiques, d'une part une activité inhibitrice de l'expression de la lysyl-hydroxylase et d'autre part une activité vasodilatatrice locale, temporaire et transitoire permettant, de plus, un apport énergétique et gazeux au niveau du bulbe pilaire. En particulier, ces dérivés sont des précurseurs de NO.

Les dérivés pyrimidine N-oxyde selon l'invention exercent donc, au contact de la peau et de façon séquentielle, une activité double, ce qui permet de les utiliser avantageusement comme actif pour stimuler et/ou induire la croissance des cheveux et/ou diminuer leur chute et/ou augmenter leur densité.

Les inventeurs ont, en outre, trouvé que ces dérivés pouvaient avoir un effet bénéfique sur la pousse des cils mais aussi de certains poils humains. Ces dérivés ont ,en outre, un effet bénéfiques sur la peau, les lèvres et les ongles.

La présente invention a donc pour objet un dérivé pyrimidine N-oxyde de formule (A) ou d'un de ses sels : 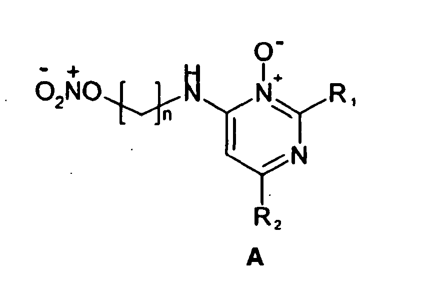 dans laquelle :
- n représente un entier allant de 2 à 12,
- R₁ représente un radical alkyle, linéaire ou ramifié, saturé ou insaturé, éventuellement substitué par un groupe -OR', -NR'R" ou -COOR', R₁ ayant de 1 à 20 atomes de carbone et mieux de 1 à 10 atomes de carbone, ou représente NR'R",
- R₂ représente l'hydrogène -NR₃R₄, -OR₃, -SR₃, avec R₃ et R₄ représentant indépendamment un radical alkyle, linéaire ou ramifié, saturé ou insaturé, éventuellement substitué par un groupe -OR', -NR'R", -COOR', R₂ ayant de 1 à 20 atomes de carbone et mieux de 1 à 10 atomes de carbone, R₃ et R₄ pouvant également faire partie d'un cycle de 4 à 7 atomes, saturé ou insaturé, contenant éventuellement au moins un hétéroatome,
- avec R' et R" égaux ou différents représentant l'hydrogène ou un radical alkyle entre C₁-C₃, linéaire ou ramifié, saturé.

L'invention a aussi pour objet une composition contenant dans un milieu physiologiquement acceptable une quantité efficace d'au moins un composé de formule (A) ou de l'un de ses sels, tel que défini précédemment. Cette composition est en particulier destinée à induire et/ou stimuler la croissance des fibres kératiniques et/ou freiner leur chute et/ou augmenter leur densité.

Aussi, l'invention se rapporte encore à l'utilisation d'au moins un composé de formule (A) ou de l'un de ses sels, tel que défini précédemment, comme agent pour induire et/ou stimuler la croissance des fibres kératiniques des êtres humains et/ou freiner leur chute et/ou augmenter leur densité. Par augmenter la densité des fibres kératiniques, et notamment la densité capillaire, on entend augmenter le nombre de fibres kératiniques, notamment de cheveux, par cm² de peau telle que le cuir chevelu.

L'invention se rapporte encore à l'utilisation cosmétique d'au moins, un composé de formule (A) ou de l'un de ses sels dans une composition cosmétique de soin et/ou de maquillage des fibres kératiniques d'être humain pour réduire la chute des fibres kératiniques et/ou augmenter leur densité. Elle a encore pour objet l'utilisation d'au moins un composé de formule (A) ou de l'un de ses sels pour la préparation d'une composition de soin et/ou de traitement des fibres kératiniques d'être humain, destinée à induire et/ou stimuler la croissance des fibres kératiniques et/ou freiner leur chute et/ou augmenter leur densité.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

En particulier, l'invention se rapporte à l'utilisation cosmétique d'au moins un composé de formule (A) ou de l'un de ses sels dans une composition cosmétique de soin capillaire d'être humain, comme agent pour traiter l'alopécie d'origine naturelle et en particulier l'alopécie androgénique ou andro-chrono-génétique, ou à l'utilisation d'au moins un composé de formule (A) ou de l'un de ses sels, pour la préparation d'une composition capillaire d'être humain, destinée à traiter l'alopécie d'origine naturelle et en particulier androgénique ou andro-chrono-génétique. Ainsi, cette composition permet de maintenir en bon état la chevelure et/ou lutter contre la chute naturelle des cheveux en particulier des hommes.

L'invention a encore pour objet l'utilisation cosmétique d'au moins un dérivé de formule (A) ou de l'un de ses sels, tel que défini ci-dessus, dans une composition cosmétique de soin et/ou de maquillage des cils d'être humain ou pour la préparation d'une composition de soin et/ou de traitement des cils d'être humain, pour ou destinée à induire et/ou stimuler la croissance des cils et/ou augmenter leur densité. Cette composition permet ainsi de maintenir en bon état les cils et/ou améliorer leur état et/ou leur aspect.

L'invention se rapporte à l'utilisation d'au moins un composé de formule (A) ou de l'un de ses sels, comme agent précurseur de NO. Elle a encore pour objet l'utilisation d'au moins un composé de formule (A) ou de l'un de ses sels, pour la fabrication d'une composition destinée à traiter les désordres liés à une réduction de la microcirculation ou vascularisation cutanée et notamment d'un follicule pileux chez l'être humain.

L'invention se rapporte à l'utilisation d'au moins un composé de formule (A) ou l'un de ses sels comme inhibiteur de lysyl-hydroxylase, et plus spécifiquement comme précurseur d'inhibiteur de lysyl-hydroxylase, ainsi que l'utilisation de ce composé (A) ou un de ses sels pour la fabrication d'une composition destinée à traiter les désordres liés à la synthèse et/ou libération de lysyl-hydroxylase.

Comme désordres liés à une réduction de la microcirculation et/ou à la synthèse et/ou libération de lysyl-hydroxylase, on peut citer le dépôt de collagène, le psoriasis, les ulcères chroniques, l'accroissement de cellulite et/ou de graisse, le syndrome de Raynaud. Ainsi, les dérivés de formule (A) peuvent être utilisés pour la préparation de composition anti-âge, amincissante ou conférant à la peau un éclat du teint ou une luminosité (appelé effet bonne mine) ou aux lèvres de la couleur ou la préparation d'une composition destinée à lutter contre le syndrome de Raynaud, les ulcères ou le psoriasis. Cette composition permet notamment un lissage et/ou une diminution des rides.

Aussi, l'invention a pour objet l'utilisation cosmétique d'au moins un dérivé de formule (A) ou l'un de ses sels, dans une composition cosmétique anti-âge, anti-ride, amincissante ou conférant à la peau un éclat du teint ou une luminosité du teint ou aux lèvres de la couleur, et l'utilisation d'au moins un composé de formule (A) ou l'un de ses sels, pour la préparation d'une composition physiologiquement acceptable, destinée à traiter les signes du vieillissement intrinsèques et/ou extrinsèques, les surcharges pondérales, les ulcères, le syndrome de Raynaud et/ou le psoriasis et/ou améliorer la cicatrisation cutanée. Les signes du vieillissement extrinsèques sont en particulier ceux dus aux rayonnements ultraviolets (lampes U.V. ou soleil intense).

Par leur activité précurseur de NO, les dérivés selon l'invention permettent aussi de lutter contre les mycoses cutanées, les désordres inflammatoires comme les érythèmes notamment solaires ou de lupus, les réactions d'hypersensibilité de contact et/ou les manifestations allergiques, l'eczéma, les prurits, les peaux sensibles, y compris le cuir chevelu sensible, mais aussi contre les hypermélanoses.

Aussi, l'invention a encore pour objet l'utilisation d'au moins un composé de formule (A) ou l'un de ses sels, pour la fabrication d'une composition physiologiquement acceptable, destinée à traiter les mycoses cutanées, les désordres inflammatoires, les réactions d'hypersensibilité de contact, les manifestations allergiques, l'eczéma, les prurits, les peaux sensibles et/ou l'hypermélanose.

La présente invention se rapporte également à un procédé de traitement cosmétique des fibres kératiniques humaines et/ou de la peau y compris le cuir chevelu, destiné notamment à stimuler la croissance des fibres kératiniques humaines telles que les cheveux et les cils d'être humain et/ou freiner leur chute, caractérisé par le fait qu'il consiste à appliquer sur les fibres kératiniques humaines et/ou la peau, une composition cosmétique comprenant une quantité efficace d'au moins un composé de formule (A) ou d'un de ses sels, à laisser celle-ci en contact avec les fibres kératiniques et/ou la peau, et éventuellement à rincer les fibres kératiniques et/ou la peau.

Ce procédé de traitement présente les caractéristiques d'un procédé cosmétique dans la mesure où il permet d'améliorer l'esthétique des fibres kératiniques et en particulier des cheveux et des cils en leur donnant une plus grande vigueur et un aspect amélioré. En outre, il peut être utilisé quotidiennement pendant plusieurs mois, sans prescription médical.

Plus spécialement, la présente invention se rapporte à un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant au moins un dérivé de formule (A) ou l'un de ses sels, à laisser celle-ci au contact des cheveux et/ou du cuir chevelu, et éventuellement à rincer les cheveux et/ou le cuir chevelu.

L'invention se rapporte encore à un procédé de soin cosmétique et/ou de maquillage ces cils humains, en vue d'améliorer leur état et/ou leur aspect, caractérisé en ce qu'il consiste à appliquer une composition de mascara comprenant au moins un composé de formule (A) ou l'une de ses sels et à laisser celle-ci au contact des cils. Cette composition de mascara peut être appliquée seule ou en sous-couche d'un mascara pigmenté classique et être éliminée comme un mascara pigmenté classique.

L'invention a encore pour objet un procédé de fabrication d'un dérivé de pyrimidine N-oxyde défini précédemment, comprenant les étapes suivantes :
1°) Réaction du composé **5** 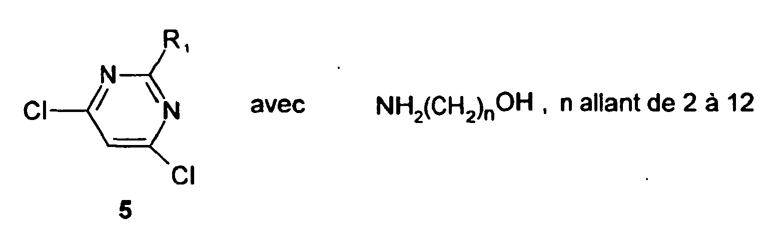
2°) Oxydation du produit obtenu à l'étape 1°),
3°) Substitution du produit obtenu à l'étape 2°) pour substituer un atome de chlore par un substituant,
4°) Nitration du produit obtenu à l'étape 3°).

En particulier, la substitution est une hydrogénation permettant de remplacer l'atome de chlore par un atome d'hydrogène.

### DESCRIPTION DETAILLEE DES MODES DE REALISATION DE L'INVENTION

Avantageusement, les composés de formule (A), sous forme salifiée ou non, présentent une activité double, exercée de façon séquentielle au niveau de la peau et en particulier du cuir chevelu. Les composés A ont la particularité d'être inactifs, en l'absence d'enzyme et en particulier d'hydrolases.

Autrement dit, les composés A inactifs, libèrent du NO *in vivo,* sous l'action des enzymes cutanées (par exemple les hydrolases), qui se traduit par un effet bénéfique sur la microcirculation : le NO libéré ayant un effet vasodilatateur.

Les composés A' obtenus à partir de A inactif, réagissent alors comme des inhibiteurs « retard » de l'expression des enzymes type hydroxylase et en particulier de la lysyl hydroxylase (noté LH).

Le schéma réactionnel est le suivant : 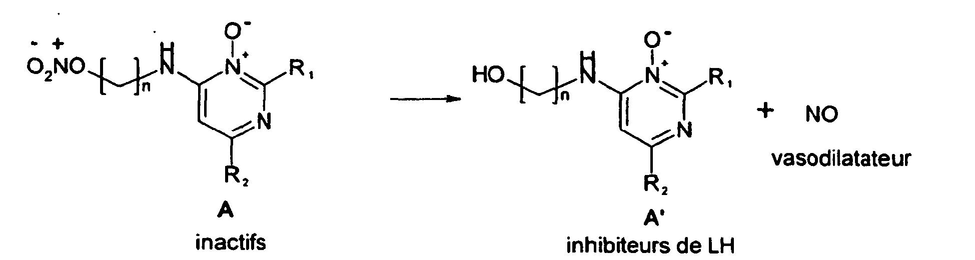 avec n, R₁ et R₂ ayant la signification précédemment indiquée.

Dans la suite du texte, et sauf mention exprès, l'emploi du terme composé de formule (A) doit être compris comme signifiant aussi bien le composé de formule (A) sous forme acide ou basique, que l'un de ses sels. Il peut aussi être sous forme tautomère.

"Au moins un" selon l'invention signifie un ou plusieurs (2, 3 ou plus). En particulier, la composition peut contenir un ou plusieurs composés de formule (A). Ce ou ces composés peuvent être des isomères cis ou trans ou un mélange d'isomères cis/trans. Ils peuvent aussi être sous forme tautomère. Ce ou ces composés peuvent être des énantiomères et/ou des diastéréoisomères ou un mélange de ces isomères, en particulier un mélange racémique.

Selon l'invention, R₃ et R₄ peuvent faire partie seuls ou ensemble d'un cycle comportant de 4 à 7 atomes et mieux de 5 à 6 atomes. Ils peuvent être saturés ou insaturés et comporter éventuellement un ou plusieurs hétéroatomes tels que S, N, O ou leurs associations et par exemple de 1 à 4 hétéroatomes. Comme cycles carbonés saturés utilisables on peut citer le radical cyclobutyle, cyclopentyle, cyclohexyle ou cycloheptyle. Comme hétérocycle Hy, on peut citer les cycles pyridine, pipéridine, morpholine, pyrrole, furanne, thiophène, imidazole, oxazole, thiazole, pyrazole, pyrimidine, pipérazine, pyrazine, pyridazine, triazine, pyrrolidine, thiazolidine. Comme cycles carbonés insaturés, on peut citer le cycle cyclohexényle ou phényle et comme radical aryle, on peut citer le radical phényle ou naphtyle. En outre, ces cycles peuvent éventuellement être substitués en particulier par un substituant T₂ choisi parmi les halogènes et les radicaux alkyle et C₁-C₁₀, linéaires ou ramifiés.

De plus, ces cycles R₃ et R₄ peuvent être seuls ou accolés à un autre cycle de même structure chimique ou non.

Lorsque R₃ et R₄ font partie d'un hétérocycle, cet hétérocycle comporte au moins un atome d'azote comme hétéroatome et peut être, par exemple, le cycle pyrrolidine, pyrrole, imidazole, triazole, pipéridine, morpholine, pipérazine, ou tétrazole, oxazole, isoxazole, pyridazine, pyridine. De préférence R₃ et R₄ forment avec l'azote auquel ils sont liés un hétérocycle comportant au moins un atome d'azote comme hétéroatome.

Comme exemple de radical alkyle utilisable dans l'invention, on peut citer les radicaux méthyle, éthyle, isopropyle, n-buyle, terbutyle, n-hexyle.

Comme atome d'halogène, on peut utiliser les atomes de chlore, de fluor ou de brome, et mieux les atomes de fluor et de chlore.

Selon l'invention, les composés de formule (A) sont sous forme isolée, c'est-à-dire non polymérique.

Avantageusement, l'un au moins des R₁ et R₂ représente un atome d'hydrogène, un hétérocycle Hy saturé ou non.

Selon un mode de réalisation de l'invention R₁ représente NRR' avec en particulier R et R' représentant H.

Selon un autre mode de réalisation de l'invention R₂ représente NR₃R₄ avec en particulier R₃ et R₄ formant avec l'azote auquel ils sont liés un hétérocycle Hy. En particulier, cet hétérocycle est saturé et comporte 6 atomes. A titre d'exemple, R₂ représente H et R₁ représente NRR' avec R et R' valant notamment H ; R₂ représente un hétérocycle saturé à 6 atomes, l'hétéroatome étant l'azote et R₁ représente NRR' avec R et R' valant notamment H. L'hétérocycle est par exemple le cycle pipéridine.

De préférence, n est un entier allant de 2 à 8 et mieux de 2 à 4.

Par sels de composé de formule (A), on entend selon l'invention, les sels organiques ou inorganiques d'un composé de formule (A).

Comme sels inorganiques utilisables selon l'invention on peut citer : les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺) et de manganèse (Mn²⁺); les hydroxydes, les carbonates, les chlorures, les sulfates, les citrates, les acétates, les lactates.

Les sels organiques utilisables selon l'invention sont par exemple les sels de tri-éthanolamine, mono-éthanolamine, di-éthanolamine, hexadécylamine et N,N,N',N'-tétrakis-(hydroxy-propyl-2) éthylène diamine, tris-hydroxyméthylaminométhane.

A la connaissance du demandeur, aucun document de l'art antérieur ne décrit ni ne suggère que les composés de formule (A) ou leurs sels aient la propriété d'induire et/ou de stimuler la croissance des fibres kératiniques et/ou de freiner leur chute ni que ces composés peuvent être utilisés par voie topique pour augmenter la densité de ces fibres.

La quantité efficace d'un composé de formule (A) ou de l'un de ses sels correspond à la quantité nécessaire pour obtenir le résultat désiré (en particulier à savoir augmenter la densité des fibres kératiniques ou favoriser leur pousse). L'homme du métier est donc en mesure d'évaluer cette quantité efficace qui dépend de la nature du composé utilisé, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, le composé de formule (A) ou de l'un de ses sels peut être utilisé en une quantité représentant de 10⁻³ % à 5% du poids total de la composition et préférentiellement en une quantité représentant de 10⁻² % à 2% du poids total de la composition, par exemple de 0,5 % à 2 %.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau, y compris le cuir chevelu et les paupières, les phanères tels que les fibres kératiniques ou les lèvres d'êtres humains. Par "cosmétique", on entend au sens de l'invention une composition d'aspect, d'odeur et de toucher agréables.

Le composé de formule (A), salifié ou non, peut être utilisé dans une composition qui doit être ingérée, injectée ou appliquée sur la peau ou les fibres kératiniques (sur toute zone cutanée ou fibres à traiter).

Selon l'invention, le composé de formule (A) peut être utilisé par la voie orale en une quantité de 0,1 à 300 mg par jour, 5 à 10mg/j.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau, y compris le cuir chevelu, la composition peut avoir la forme d'une solution ou suspension aqueuse, alcoolique, hydro-alcoolique ou huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une dispersion ou émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse par exemple sous forme de sérum. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants du milieu physiologique de la composition selon l'invention sont ceux généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (A) ainsi que de celle des éventuels ingrédients additionnels, pour obtenir 100% en poids. En pratique la phase aqueuse représente de 5 % à 99,9% en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et co-émulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition est une dispersion de cire-dans-eau ou de cire dans huile, une huile gélifiée, un gel aqueux, pigmenté ou non.

La composition de l'invention peut comprendre, en outre, d'autres ingrédients usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les agents bloqueurs d'U.V. comme les filtres solaires, les polymères filmogènes, les actifs cosmétiques et pharmaceutiques à action bénéfique pour la peau ou les phanères et notamment les fibres kératiniques (comme les vitamines), leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %. Ces adjuvants, selon leur nature, peuvent être introduits dans la phase grasse, dans la phase aqueuse et/ou dans les vésicules lipidiques et notamment des liposomes.

Bien entendu l'homme du métier veillera à choisir les éventuels additifs complémentaires et/ou leur quantité de telle manière que les propriétés avantageuses de la composition selon l'invention, notamment à savoir l'augmentation de la densité des fibres kératiniques, ne soient pas ou substantiellement pas, altérées par l'adjonction envisagée.

Comme solvants utilisables dans l'invention, on peut citer les alcools inférieurs de C₂ à C₈ comme l'éthanol, l'isopropanol, le propylène glycol et certaines huiles cosmétiques légères comme les alcanes en C₆ à C₁₆.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, de soja, alcool ou acide gras), les huiles d'origine animale (perhydrosqualène), les huiles de synthèse (esters d'acide gras, huile de Purcellin), les huiles siliconées (polydiméthylsiloxanes linéaires ou cycliques, phényltriméthicones) et les huiles fluorées (perfluoropolyéthers). Comme cires, on peut citer les cires siliconées, les cires d'abeille, de candellila, de riz, de carnauba ou de paraffine ou de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

La composition peut contenir un autre actif additionnel que les composés de formule (A) qui peut être hydrophile et choisi parmi les protéines ou les hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides (acide de fruit, acide salicylique); ou lipophile et choisi parmi le rétinol (vitamine A) et ses dérivés notamment ester (palmitate de rétinol), le tocophérol (vitamine E) et ses dérivés notamment ester (acétate ou palmitate de tocophérol), les acides gras essentiels, les céramides, les huiles essentielles, les dérivés de l'acide salicylique comme le n-octanoyl-5 salicylique, les esters des hydroxy acides, les phospholipides comme la lécithine, leurs mélanges.

Selon un mode particulier de réalisation de l'invention, on peut associer au composé de formule (a) ou de l'un de ses sels, au moins un composé additionnel favorisant la repousse et/ou limitant la chute des cheveux. Ces composés additionnels sont notamment choisis parmi les inhibiteurs de lipoxygénase tels que décrits dans EP 0648488, les inhibiteurs de bradykinine décrits notamment dans EP 0845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP 1175890, WO 01/74307, WO 01/74313, WO 01/74314, WO 01/74315 ou WO 01/72268, leurs mélanges.

Comme autres composés additionnels favorisant la pousse du cheveu pouvant être présents dans la composition selon l'invention on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les antiandrogènes utilisables incluent notamment les inhibiteurs stéroïdiens ou non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide et les composés décrits dans les brevets US 5 411981, 5 565467 et 4 910226.

Les composés antimicrobiens ou antifongiques peuvent être choisis parmi les dérivés du sélénium, le kétoconazole, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle et la minocycline.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0770399, WO 94/06434 et FR 2268523.

Les rétinoïdes peuvent être choisis parmi l'isotrétinoïne, l'acitrétine et le tazarotène.

Comme autres composés additionnels actifs pour favoriser la pousse et/ou limiter la chute des cheveux utilisables en association avec le composé de formule (A), salifié ou non, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les hydroxy-acides, les benzophénones et l'hydantoïne, l'acide rétinoïque ; les vitamines comme la vitamine D, les analogues de la vitamine B12 et le panthoténol ; les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les antifongiques, en particulier l'octopirox et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les agents antiandrogènes tels que l'oxendolone, la spironolactone, le diéthylstilbestrol et la flutamide ; les agonistes du récepteur FP (récepteur aux prostaglandines du type F) tels que le latanoprost, le bimatoprost, le travoprost, l'unoprostone ; les inhibiteurs de 15-hydroxyprostaglandine désydrogénase ; leurs mélanges.

On peut également envisager que la composition comprenant au moins le composé de formule (A), salifié ou non soit sous forme liposomée, telle que notamment décrite dans le document WO 94/22468. Ainsi, le composé encapsulé dans les liposomes peut être délivré sélectivement au niveau du follicule pileux.

La composition selon l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un composé de formule (A), salifié ou non, le soir, garder celle-ci au contact toute la nuit et éventuellement effectuer un shampooing le matin. Ces applications peuvent être renouvelées quotidiennement pendant un ou plusieurs mois suivant les individus.

Avantageusement, dans le procédé selon l'invention, on applique sur les zones à traiter du cuir chevelu entre 5 et 500 µl d'une solution ou composition telle que définie précédemment, comprenant de 0,001% à 5 % de composé de formule (A).

### EXEMPLES

Comme exemple de composés de formule (A) utilisable dans l'invention on peut citer les composés 1 à 4, ci-après. Ces composés donnent en présence d'enzymes cutanées (par exemple hydrolases) respectivement les composés actifs **1', 2', 3'** et **4'**.

### Exemple 1 :

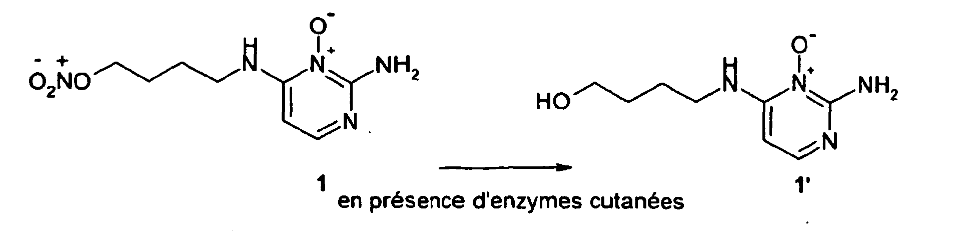

### Exemple 2 :

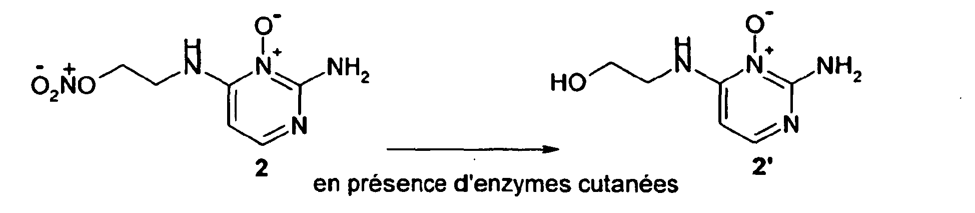

### Exemple 3:

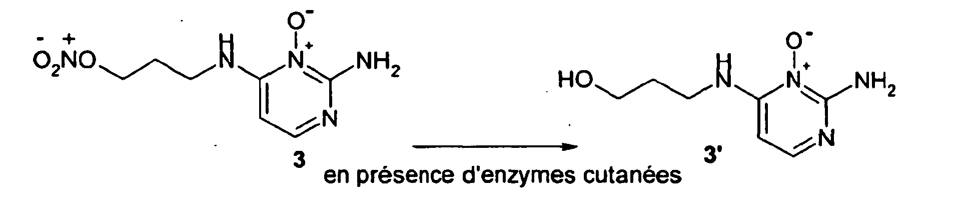

### Exemple 4 :

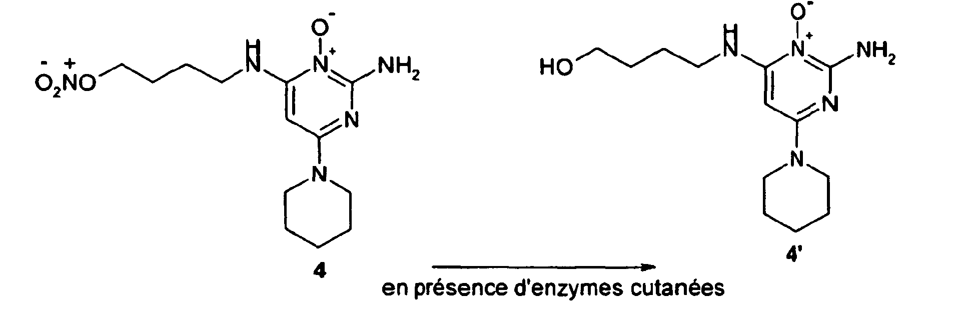

Les composés de formule (A), salifiés ou non peuvent être fabriqués de façon connue. Par exemple, la synthèse peut être réalisée selon le schéma réactionnel suivant :

### 1^{ère} étape :

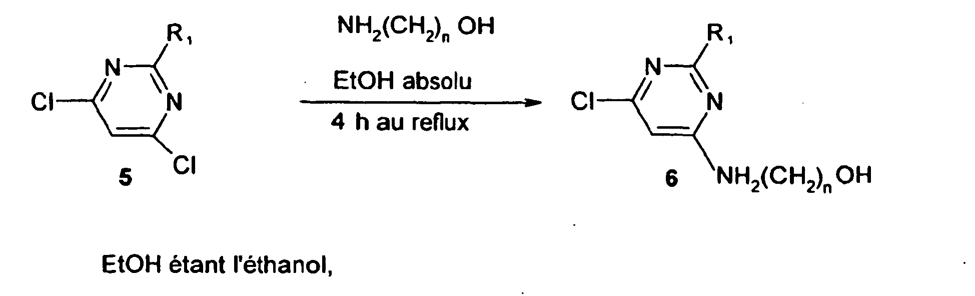

### 2^{éme} étape :

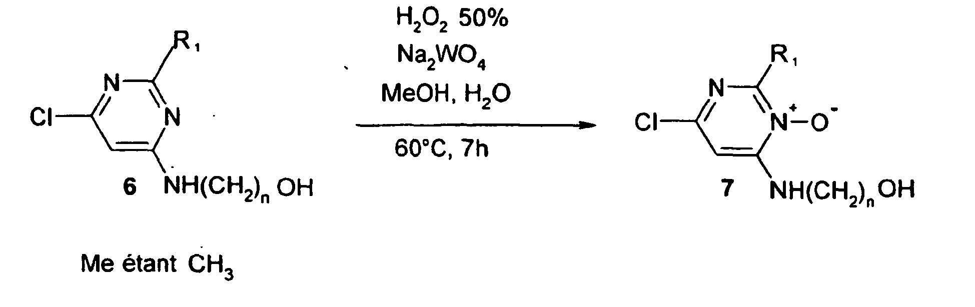

### 3^{ème} étape :

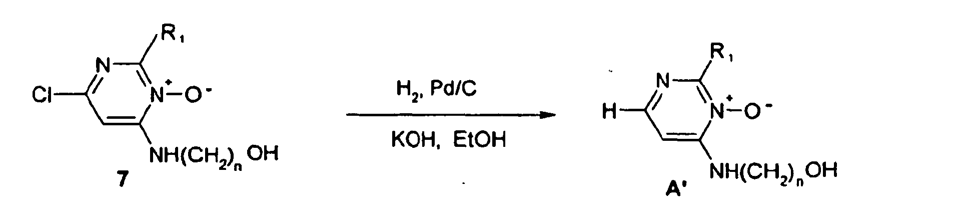

Le composé A obtenu est ensuite nitré par action avec HNO₃ fumant.

On va maintenant donner à titre d'illustration des exemples de réalisation de l'invention qui ne sauraient limiter en aucune façon sa portée.

### EXEMPLE 1 :

### Synthèse de composé 1' :

### 1^{ère} étape :

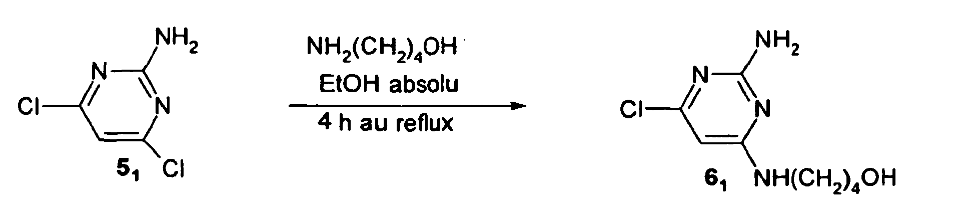

### Matières mises en oeuvre :

- 2-amino-4,6-dichloropyrimidine (composé **5₁**); 21 g, (1eq)[M=164g/mol],
- Butanolamine ; 25g, (2,2eq) [M=89g/mol],
- EtOH absolu : 150ml pour 10g.

### Mode opératoire :

Dans un flacon monocol, introduire la pyrimidine, l'éthanol absolu et le butanolamine.
Chauffer au reflux pendant 4h jusqu'à ce que tout soit solubilisé.
Evaporer le milieu ; on obtient une huile marron. Reprendre le produit dans 400 ml d'eau, laisser agiter pendant 1 h, et filtrer.
Sécher sur P₂O₅ sous vide à 50 °C.
Suivi en Chromographie en Couche Mince (CCM): CH₂Cl₂ (9) / MeOH (1), produit visible en UV et I₂. On observe une tâche plus polaire correspondant au composé 6. (coefficient de migration : Rf : 0,9).
On récupère un produit de couleur beige (M = 216,6 g/mol).
Masse récupérée : 23g ; rendement (Rdt): 85 %.

### Analyses :

Spectre de masse : conforme (M+H et M-H) à celui du composé **6₁**.
Résonance Magnétique Nucléaire (RMN) dans DMSO (diméthylsulfoxide) avec un appareil de marque Bruker 400 MHz : spectre conforme à celui du composé **6₁**.
Point de fusion mesuré par Dynamic Scanning Colorimetry (DSC) : 143-144°C.

### 2^{ème} étape :

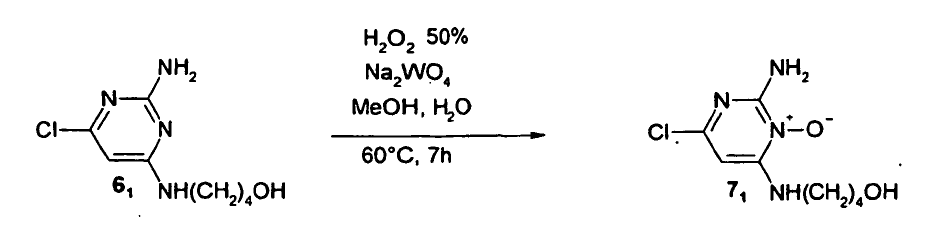

### Matières mises en oeuvre :

- Composé **6**₁ ; 23g, (1eq)[M :216,6 g/mol],
- H₂O₂ à 50% ; 14,6 ml (2,4eq) [M : 34 g/mol / d=1,19],
- Na₂WO₄: 3,5g, (0,1eq) [M : 329,86 g/mol],
- MeOH : 150ml,
- H₂O : 3 ml.

### Mode opératoire :

Dans un flacon tricol, introduire le composé **6₁** +MeOH+H₂O, puis ajouter Na₂WO₄ et 1,2 eq de H₂O₂.
Chauffer pendant 2h à 60 °C. Diminuer le chauffage et ajouter de nouveau 1,2 eq de H₂O₂.
Remettre le chauffage à 60°C pendant 5h.
Suivi en CCM : 85% CH₂Cl₂/15%MeOH/1%NH₄OH.
Révélateur des N-Oxyde : solution de FeCl₃ à 10% dans EtOH.
Laisser ensuite refroidir le milieu réactionnel et ajouter lentement une solution d'hydrogénosulfite de sodium à 37,5 % (100 ml pour 20 g) et agiter pendant 1 h puis vérifier à l'aide du papier KI qu'il n'y a plus de peroxyde.
Filtrer ensuite le milieu et évaporer. Ajouter ensuite de l'eau et extraire 3 fois avec 100 ml d'acétate d'éthyle. Extraire de nouveau la phase aqueuse (5 fois 100 ml) au butanol.
Evaporer le butanol.
On récupère un produit solide cristallin du type cire.
Masse récupérée : 10,6g ; Rdt - 50%.

### Analyses :

CCM : révèle N-oxyde (Rf : 0,56),
Spectre de masse conforme à celui du produit **7₁** (ES+= 233, ES-=231)

### 3^{ème} étape :

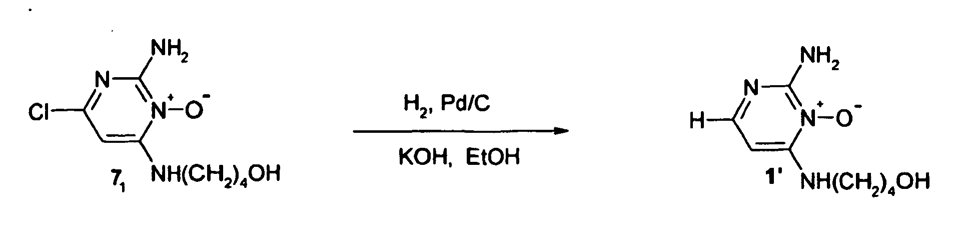

### Matières mises en oeuvre :

- Composé **7**₁ : 10,6g, (1 eq) [M=232,7 g/mol],
- KOH : 6,6g (2.2eq), [M = 56 g/mol],
- EtOH absolu : 600 ml,
- Pd/C à 10 %, sec : 900 mg.

### Mode opératoire :

Mélanger la potasse et l'alcool puis le composé **7₁** jusqu'à ce que tout soit solubilisé et au bout d'une heure filtrer. Ajouter le Pd/C, et hydrogéner avec une pression d'H₂ = 10 bars (10⁶ Pa) à T° ambiante. Filtrer sur célite, ramener à pH = 4-5 avec HCl 35%. Filtrer la partie insoluble et évaporer. Le produit est récupéré dans de l'éthanol puis on ajoute de l'acétone (30ml /75 ml). Filtrer.
Le produit obtenu est blanc cassé.
Masse récupérée : 4g, Rendement - 45% ; [M=198,2 g/mol].

### Analyses :

CCM : 80% CH₂Cl₂ /19%MeOH/1%NH₄OH. (Rf : 0,38),
Spectre de masse conforme à celui du composé **1**',
RMN proton 400 MHz dans DMSO : conforme à celui du produit **1'**,
Point de fusion mesuré par DSC : 164-165°C.

### Résultats biologiques :

### Activité donneurs de NO du Composé 1 par rapport au composé 1' :

L'activité des composés **A** et **A'** sur la NO-synthase inductible a été évaluée dans le test décrit par Heck et col. (J.B.C., Vol. 267, N° 30, 21277-21280, 25 octobre 1992), test de « Modulation de l'induction de la NOS₂ (Nitric Oxide Synthase inductible) sur kératinocytes humains normaux». Ce test a pour objectif d'évaluer la concentration en nitrates et nitrites, in fine, après stimulation de la NO-synthase 2 ou application d'un donneur de NO ; il est réalisé par comparaison avec la stimulation de la NO-synthase inductible par des cytokines. La concentration en nitrates et en nitrites, in fine, correspond à la concentration de NO libéré.

Le test est effectué sur une culture de kératinocytes humains normaux, issus de prélèvements. L'induction de la NO-synthase inductible (NOS₂) a été provoquée par l'addition d'une combinaison de plusieurs cytokines au milieu de culture. Les produits testés ont été appliqués à trois concentrations variant de 10 à 1000 µM.

Les contrôles suivants ont été introduits dans le test :
A : contrôle positif (induction de l'enzyme sur cellules stimulées) : mélanges d'interféron-γ (1000 µ/ml) et d'interleukine 1-β (100 µ/ml) correspondant à 100% d'inhibition ;
B : contrôle basal des cellules non stimulées (sans cytokines), correspondant à 0% d'inhibition.

Pour déterminer l'activité des produits à tester, on mesure la quantité de produits de réaction stables du NO (nitrites et nitrates) à l'aide du kit "Nitric ColorimetricAassay" vendu par la société Boehringer sous la référence 1756.28.

Les composés ont été tests aux concentrations de 100 µM, 500 µM et 1 000 µM dans l'éthanol.

| **Produit testé** | **Concentration** | **% inhibition** |
|---|---|---|
| A | | 100 |
| B | | 0 |
| **1** | 100 µM | 0 |
| **1** | 500 µM | 18,23 % |
| **1** | 1000 µM | 192,47 % |
| **1'** | 500 µm | SE* |
| **1'** | 1000 µm | SE* |
| **2** | 100 µM | 38,77 |
| **2** | 500 µM | 292,57 |
| **2** | 1000 µm | 355,2 |
| **2'** | 500 µm | SE |
| **2'** | 1000 µm | SE |
| **3** | 100 µM | 34043 |
| **3** | 500 µm | 296,17 |
| **3** | 1000 µm | 361,17 |
| **3'** | 500 µm | SE * |
| **3'** | 1000µm | SE * |

| | | |
|---|---|---|
| *SE signifie : sans effet | | |

Du tableau ci-dessus, on conclut que les composé **1, 2, 3** présentent un effet donneur de NO à l'inverse des produits **1', 2', 3'**.

### Activité inhibition de l'expression de LH :

### a) Test

Des fibroblastes dermiques humains normaux (NHDF) sont précultivés à très forte densité pendant 48heures dans du milieu MEM199 (Gibco) supplémenté avec 1% de sérum de veau foetal puis traités ou non par le composé **1'** ou **1** pendant 6 heures. Les tapis cellulaires sont ensuite lavés à l'aide d'un tampon salin phosphate (PBS) puis congelés en milieu Tri-reagent (Sigma). Les ARN totaux obtenus sont extraits à l'aide de Tri-reagent (Sigma) selon le protocole du fournisseur. Une nouvelle extraction est réalisée au chloroforme puis les ARN sont précipités à l'isopropanol. Après élimination des traces d'ADN contaminant par traitement avec le système DNA-free (Ambion), une reverse transcription de l'ARNs en ADN complémentaire est réalisée en présence d'oligo dT et de l'enzyme superscript II (Gibco).

Un couple d'amorces (primers) permettant l'amplification de fragments spécifiques LH (516bp ; Mahé et al 1996, Skin Pharmacol. 9, 177-183) et des amorces spécifiques de séquence du collagène l (420pb) et β-Actine (500pb, standard de réaction interne) ont été utilisés pour la réaction de polymérisation en chaîne (PCR) avec le système PCR Supermix (Gibco) dans les conditions suivantes : 94°C, pendant 2min puis 25 fois (95°C-1min, 55°C-1 min, 72°C-2min) la réaction en chaîne étant terminée par un cycle d'élongation de 7 min à 72C.
Les fragments amplifiés sont ensuite analysés par électrophorèse sur gel d'agarose (1,5%) en présence de bromure d'ethidium. La saisie des images a été réalisée sur GelPrint 2000i (Biophotonics Corp) ; les analyses densitométriques ont été obtenues à l'aide du logiciel One D-Scan (Scanalytics).

Les résultats sont exprimés en pourcentage d'expression de LH sur le standard interne β-Actine (LH/actine) ou par rapport à l'ARN messager du collagène (LH/collagène). Une baisse de l'un ou des deux pourcentages indique une inhibition transcriptionnelle de l'expression relative de la lysyl hydroxylase et, par analogie avec la molécule 2-4 DPO, (Mahé et al 1996, Skin Pharmacol. 9, 177-183) une utilisation possible pour limiter la réticulation anormale du collagène dans l'alopécie.

| | Ratio LH/Actine | Ratio LH/Collagène |
|---|---|---|
| Témoin | 100 | 100 |
| Composé 1 | 123 | 133 |
| Composé 1' | 64 | 76 |

### b) Résultats :

Le dérivé **1** ne montre pas d'activité inhibitrice d'expression des ARN messagers de la lysyl hydroxylase. Le composé **1'** à 10µM possède en revanche cette capacité d'inhiber de -24% à -36% l'expression de la lysyl hydroxylase par des fibroblastes humains en culture.

### EXEMPLE 2 :

### Synthèse de composé 2' :

### 1^{ère} étape :

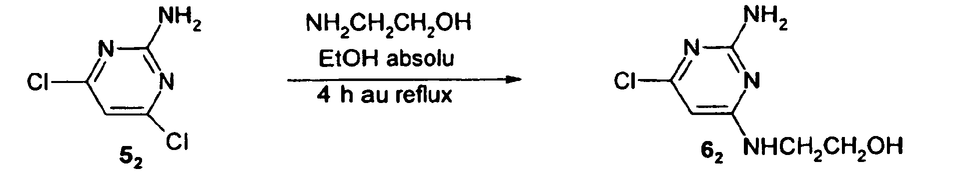

### Matières mises en oeuvre :

- 2-amino-4,6-dichloropyrimidine (composé **5₂**) : 20g, (1eq) [M=164g/mol],
- Ethanolamine : 16,3g, (2,2eq) [M=61g/mol / d=1,016],
- EtOH absolu : 300ml.

### Mode opératoire:

Dans un flacon monocol, introduire le composé **5**₂, l'éthanol absolu et l'éthanolamine. Chauffer au reflux pendant 4h jusqu'à ce que tout soit solubilisé. Evaporer le milieu ; on obtient une huile marron, qu'on solubilise dans 200ml d'acide acétique, puis on ajoute 300 ml d'eau. Extraire ensuite 6 fois à l'acide acétique. Sécher les phases organiques, sur Na₂SO₄ anhydre puis évaporer.
Reprendre le produit dans de l'éther isopropylique, agiter pendant 1 h, filtrer. Sécher sous vide à 50 °C.
Suivi en CCM : CH₂Cl₂ (9) / MeOH (1), produit visible en UV et I₂ ; la tâche plus polaire correspond au composé **6₂** ; Rf : 0,24. Masse récupérée : 17g ; Rdt 75 %.
Produit de couleur beige [M = 188,6 g/mol].

### Analyses :

Spectre de masse : conforme (M+H et M-H) à celui du composé **6₂**. Spectre RMN dans DMSO 400 MHz : conforme à celle du produit **6₂**.
Point de fusion mesuré par DSC : 150-151°C.

### 2ème étape :

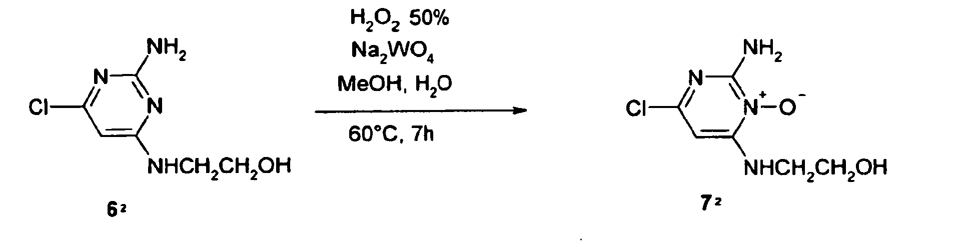

### Matières mises en oeuvre :

- Composé **6₂** : 17g, (1eq) [M :188,6 g/mol],
- H₂O₂ à 50% : 12,4 ml (2,4eq), [M : 34 g/mol / d=1,19],
- Na₂WO₄: 3g (0,1eq) [M : 329,86 g/mol],
- MeOH : 100ml,
- H₂O : 3 ml,

### Mode opératoire :

Dans un flacon tricol, introduire le composé **6**₂ +MeOH+H₂O, ajouter Na₂WO₄ et 1,2 eq de H₂O₂.
Chauffer pendant 2h à 60 °C. Diminuer le chauffage et ajouter de nouveau 1,2 eq de H₂O₂.
Remettre le chauffage à 60°C pendant 5h.
Suivi en CCM : CH₂Cl₂ 85% /MeOH 15%/NH₄OH 1%.
Révélateur de N-Oxyde : solution de FeCl₃ à 10% dans EtOH. (tache marron), Rf: 0,33.
Laisser ensuite refroidir le milieu réactionnel et ajouter lentement une solution d'hydrogénosulfite de sodium à 37,5 % (100 ml pour 20 g) et laisser agiter pendant 1h puis vérifier à l'aide du papier KI qu'il n'y a plus de peroxyde. Filtrer les parties insolubles, ensuite le milieu réactionnel et évaporer. Ajouter ensuite de l'eau et extraire 3 fois avec 100 ml d'acétate d'éthyle. On extrait de nouveau la phase aqueuse (5 fois 100 ml) de butanol. Evaporer le butanol. On récupère une pâte.
Masse récupérée : 10,5 g, Rdt ~ 50%.

### Analyses :

CCM : révèle N-oxyde

### 3^{ème} étape:

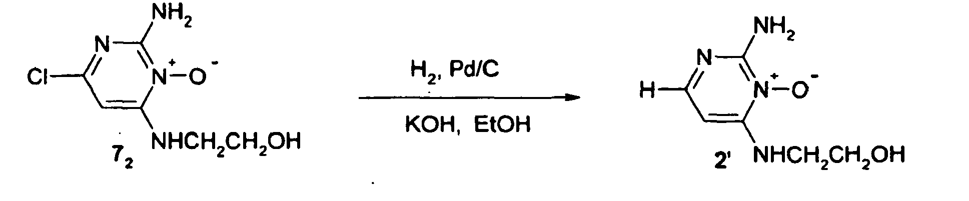

### Matières mises en oeuvre :

- Composé **7₂** : 10,5g, (1 eq), [M=204,6 g/mol],
- KOH : 8g (2,2eq), [M = 56 g/mol],
- EtOH absolu : 600 ml,
- Pd/C à 10 % sec : 1,2g.

### Mode opératoire :

Mélanger la potasse et l'alcool puis le composé **7₂** jusqu'à ce que tout soit solubilisé, et au bout d'une heure filtrer. Ajouter le Pd/C, et hydrogéner avec une pression d'H₂ = 10 Bars (10⁶ Pa) à T° ambiante. Filtrer sur célite, ramener à pH = 4-5 avec HCl 35%. Filtrer les parties insolubles et évaporer. Le produit est récupéré dans de l'éthanol que l'on chauffe puis on ajoute de l'acétonitrile (15ml /100 ml). Laisser revenir à T° ambiante puis filtrer.

On obtient un produit blanc cassé.
Masse récupérée : 2,4g, Rendement ~ 30% ; M=170,2 g/mol.

### Analyses :

CCM : 80% CH₂Cl₂/19% MeOH/1% NH₄OH, (Rf : 0,3).
Spectre de masse conforme à celui du composé **2**'.
Spectre RMN proton 400 MHz dans DMSO ; conforme à celui du produit **2**'.
Point de fusion : 186-187°C.

### EXEMPLES 3 et 4 :

### Synthèse de composé 3':

Le composé **3'** a été préparé par la même voie de synthèse et les mêmes modes opératoires que ceux des composés **1'** et **2'.**

### Synthèse de composé 4' :

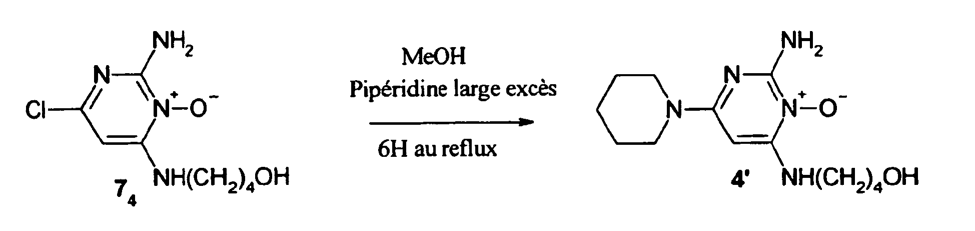

- Composé **7₄** : 1,5 g, (M=232,6 g/mol),
- Pipéridine : 5 ml, (M=8,15 g/mol),
- MeOH : 30 ml.

### Mode opératoire :

Dans un réacteur introduire le composé **7**₄ avec 30 ml de MeOH, ajouter 2 ml de pipéridine et porter à reflux. Au bout de 3 h de chauffage rajouter 3 ml de pipéridine et laisser au reflux de nouveau 3 h. Il n'y a plus de produit de départ sur plaque. Suivi en CCM : CH₂Cl₂ 85% / MeOH 15% / NH₄OH 1%.
Concentrer le milieu réactionnel, puis reprendre le résidu avec de l'eau. Faire un lavage au dichlorométhane et 3 extractions au butanol. Evaporer le butanol. Obtention d'un résidu mi-solide, mi-liquide. Reprendre ce dernier dans 10 ml d'acétonitrile sous agitation. Un solide se disperse finement. Après une nuit sous agitation, on filtre et on rince par 2 fois avec 15 ml d'acétone. Sécher au dessiccateur sous vide.
Masse récupérée : 0,250g ; rdt 14 % (M=281,35 g/mol).

### Analyses :

Spectre RMN proton dans DMSO à 200 MHz : conforme à celle du composé **4**'.

### Méthode de nitration général des molécules :

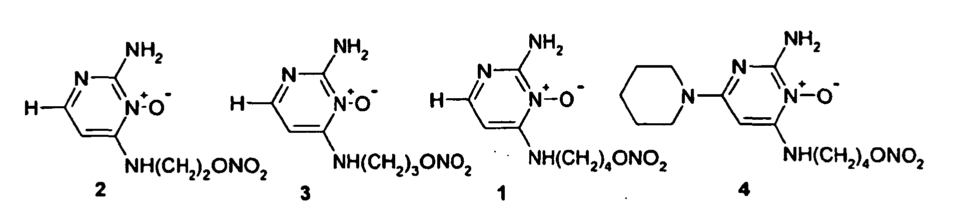

### Matières mises en oeuvre :

- Produit de départ : 1 g,
- HNO₃ fumant : 5 ml.

### Mode opératoire :

Dans un ballon introduire HNO₃ fumant et amener la température à 0°C. Ajouter petit à petit le produit de départ. Laisser sous agitation pendant 1 h à 0°C. Suivi en CCM : 80% CH₂Cl₂ /19% MeOH/1% NH₄OH. Verser le milieu réactionnel dans un mélange eau/glace (≈ 30 ml). Ramener le pH à 9, avec une solution de NaOH à 35 %. Extraire au butanol, évaporer et reprendre dans 5 ml d'acétone. Sécher au dessiccateur sous vide à T° ambiante.

### Analyses :

Spectre RMN proton 400 MHz dans DMSO conformes à ceux des composés **1** à **4**.

Les compositions ci-après sont obtenues par les techniques habituelles couramment utilisées dans le domaine cosmétique ou pharmaceutique.

### EXEMPLE 5 : Lotion capillaire

- Composé 1 1,00 g
- Propylène glycol 30,00 g
- Alcool éthylique 40,00 g
- Eau qsp 100,00 g

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse.

## Revendications

1. Dérivé pyrimidine de N-oxyde de formule (A) ou d'un de ses sels : 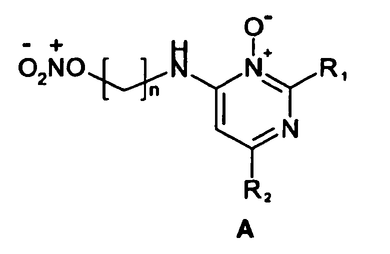 dans laquelle :
- n représente un entier allant de 2 à12,
- R₁ représente un radical alkyle, linéaire ou ramifié, saturé ou insaturé, éventuellement substitué par un groupe -OR', -NR'R" ou -COOR', R₁ ayant de 1 à 20 atomes de carbone et mieux de 1 à 10 atomes de carbone, ou représente NR'R",
- R₂ représente l'hydrogène -NR₃R₄, -OR₃, -SR₃, avec R₃ et R₄ représentant indépendamment un radical alkyle, linéaire ou ramifié, saturé ou insaturé, éventuellement substitué par un groupe -OR', -NR'R", -COOR', R₂ ayant de 1 à 20 atomes de carbone et mieux de 1 à 10 atomes de carbone, R₃ et R₄ pouvant également faire partie d'un cycle de 4 à 7 atomes, saturé ou insaturé, contenant éventuellement au moins un hétéroatome,
- avec R' et R" égaux ou différents représentant l'hydrogène ou un radical alkyle entre C₁-C₃, linéaire ou ramifié, saturé.

2. Dérivé selon la revendication précédente, **caractérisée en ce que** n est un entier allant de 2 à 6 et mieux de 2 à 4.

3. Dérivé selon l'une des revendications précédentes, **caractérisé en ce que** l'un au moins des R₁ et R₂ représentent un hydrogène.

4. Dérivé selon l'une des revendications précédentes, **caractérisé en ce que** R₁ représente NRR'.

5. Dérivé selon la revendication précédente, **caractérisée en ce que** R et R' représentent l'hydrogène.

6. Dérivé selon l'une des revendications précédentes, **caractérisé en ce que** R₂ représente NR₃R₄.

7. Dérivé selon l'une des revendications précédentes, **caractérisé en ce que** R₃ et R₄ font partie d'un hétérocycle.

8. Dérivé selon l'une des revendications précédentes, **caractérisé en ce que** R₃ et R₄ forment avec l'azote auquel ils sont liés un hétérocycle.

9. Dérivé selon l'une des revendications précédentes, **caractérisé en ce que** R₂ représente l'hydrogène et R₁ représente NRR' avec R et R' valant H ou **en ce que** R₂ représente un hétérocycle saturé à 6 atomes, l'hétéroatome étant l'azote et R₁ représente NRR' avec R et R' valant H.

10. Dérivé selon l'une des revendications précédentes, **caractérisé en ce que** le sel du dérivé de formule (A) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), les sels de tri-éthanolamine, mono-éthanolamine, di-éthanolamine, hexadécylamine, N,N,N',N'-tétrakis-(hydroxy-propyl-2) éthylène di-amine, tris-hydroxyméthylaminométhane, les hydroxydes, les carbonates et les chlorures, les sulfates, les citrates, les acétates, les lactates.

11. Dérivé selon l'une des revendications précédentes, **caractérisé en ce que** le dérivé satisfait à l'une des formules suivantes :

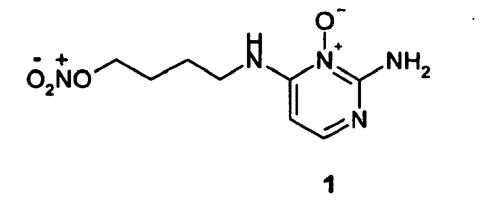

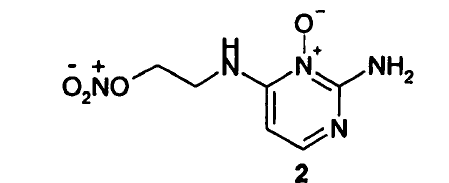

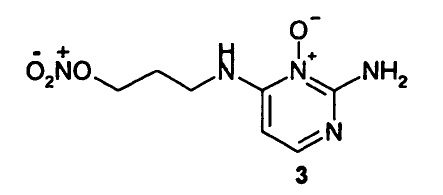

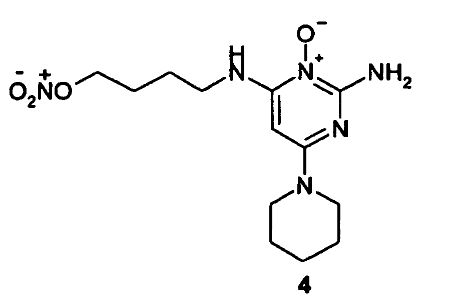

12. Utilisation d'au moins un dérivé de pyrimidine N-oxyde de formule (A) ou de l'un de ses sels, 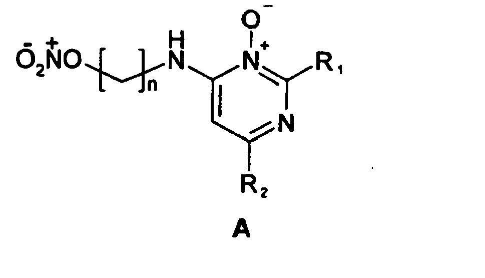 dans laquelle :
- n représente un entier allant de 2 à 12
- R₁ représente un radical alkyle, linéaire ou ramifié, saturé ou insaturé, éventuellement substitué par un groupe -OR', -NR'R" ou -COOR', R₁ ayant de 1 à 20 atomes de carbone et mieux de 1 à 10 atomes de carbone, ou représente NR'R"
- R₂ représente l'hydrogène -NR₃R₄, -OR₃, -SR₃, avec R₃ et R₄ représentant indépendamment un radical alkyle, linéaire ou ramifié, saturé ou insaturé, éventuellement substitué par un groupe -OR', -NR'R", -COOR', R₂ ayant de 1 à 20 atomes de carbone et mieux de 1 à 10 atomes de carbone, R₃ et R₄ pouvant également faire partie d'un cycle de 4 à 7 atomes, saturé ou insaturé, contenant éventuellement au moins un hétéroatome,
- avec R' et R" égaux ou différents représentant l'hydrogène ou un radical alkyle entre C₁-C₃, linéaire ou ramifié, saturé,
pour la fabrication d'une composition destinée à traiter les désordres liés à la synthèse et/ou libération de lysyl-hydroxylase et/ouliés à la réduction de la microcirculation cutanée.

13. Utilisation d'au moins un dérivé de pyrimidine N-oxyde de formule (A) ou de l'un de ses sels, 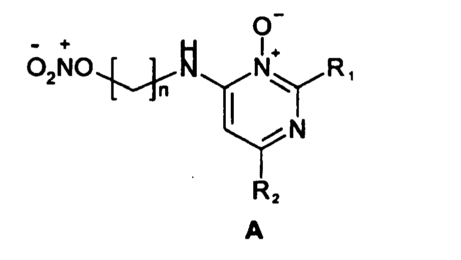 dans laquelle :
- n représente un entier allant de 2 à 12
- R₁ représente un radical alkyle, linéaire ou ramifié, saturé ou insaturé, éventuellement substitué par un groupe -OR', -NR'R" ou -COOR', R₁ ayant de 1 à 20 atomes de carbone et mieux de 1 à 10 atomes de carbone, ou représente NR'R"
- R₂ représente l'hydrogène -NR₃R₄, -OR₃, -SR₃, avec R₃ et R₄ représentant indépendamment un radical alkyle, linéaire ou ramifié, saturé ou insaturé, éventuellement substitué par un groupe -OR', -NR'R", -COOR', R₂ ayant de 1 à 20 atomes de carbone et mieux de 1 à 10 atomes de carbone, R₃ et R₄ pouvant également faire partie d'un cycle de 4 à 7 atomes, saturé ou insaturé, contenant éventuellement au moins un hétéroatome,
- avec R' et R" égaux ou différents représentant l'hydrogène ou un radical alkyle entre C₁-C₃, linéaire ou ramifié, saturé,
pour la préparation d'une composition de soin et/ou de traitement des fibres kératiniques humaines, destinée à induire et/ou stimuler la croissance des fibres kératiniques humaines et/ou freiner leur chute et/ou augmenter leur densité.

14. Utilisation d'au moins un dérivé de pyrimidine N-oxyde de formule (A) ou de l'un de ses sels, 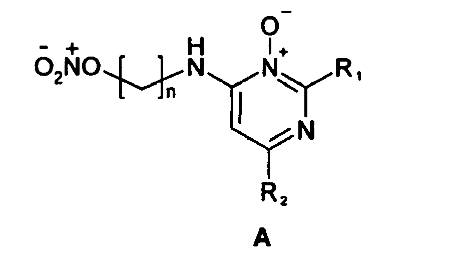 dans laquelle :
- n représente un entier allant de 2 à 12,
- R₁ représente un radical alkyle, linéaire ou ramifié, saturé ou insaturé, éventuellement substitué par un groupe -OR', -NR'R" ou -COOR', R₁ ayant de 1 à 20 atomes de carbone et mieux de 1 à 10 atomes de carbone, ou représente NR'R"
- R₂ représente l'hydrogène -NR₃R₄, -OR₃, -SR₃, avec R₃ et R₄ représentant indépendamment un radical alkyle, linéaire ou ramifié, saturé ou insaturé, éventuellement substitué par un groupe -OR', -NR'R", -COOR', R₂ ayant de 1 à 20 atomes de carbone et mieux de 1 à 10 atomes de carbone, R₃ et R₄ pouvant également former ensemble un cycle de 4 à 7 atomes, saturé ou insaturé, contenant éventuellement au moins un hétéroatome,
- avec R' et R" égaux ou différents représentant l'hydrogène ou un radical alkyle entre C₁-C₃, linéaire ou ramifié, saturé,
pour la préparation d'une composition physiologiquement acceptable, destinée à traiter les signes du vieillissement intrinsèques et extrinsèques, les surcharges pondérales, les ulcères, le syndrome de Raynaud, le psoriasis, les mycoses cutanées, les désordres inflammatoires, les réactions d'hypersensibilité de contact, les manifestations allergiques, l'eczéma, les prurits, les peaux sensibles, l'hypermélanose et/ou améliorer la cicatrisation cutanée.

15. Utilisation d'au moins un dérivé de pyrimidine N-oxyde de formule (A) ou d'un de ses sels, 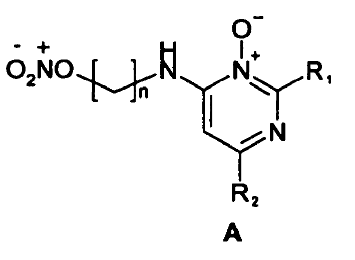 dans laquelle :
- n représente un entier allant de 2 à 12,
- R₁ représente un radical alkyle, linéaire ou ramifié, saturé ou insaturé, éventuellement substitué par un groupe -OR', -NR'R" ou -COOR', R₁ ayant de 1 à 20 atomes de carbone et mieux de 1 à 10 atomes de carbone, ou représente NR'R"
- R₂ représente l'hydrogène -NR₃R₄, -OR₃, -SR₃, avec R₃ et R₄ représentant indépendamment un radical alkyle, linéaire ou ramifié, saturé ou insaturé, éventuellement substitué par un groupe -OR', -NR'R", -COOR', R₂ ayant de 1 à 20 atomes de carbone et mieux de 1 à 10 atomes de carbone, R₃ et R₄ pouvant également faire partie d'un cycle de 4 à 7 atomes, saturé ou insaturé, contenant éventuellement au moins un hétéroatome,
- avec R' et R" égaux ou différents représentant l'hydrogène ou un radical alkyle entre C₁-C₃, linéaire ou ramifié, saturé,
pour la fabrication d'une composition capillaire d'être humain, destinée à induire et/ou stimuler la croissance des cheveux et/ou freiner leur chute et/ou augmenter leur densité et/ou traiter l'alopécie androgénique.

16. Utilisation d'au moins un dérivé de pyrimidine de N-oxyde de formule (A) ou de l'un de ses sels, 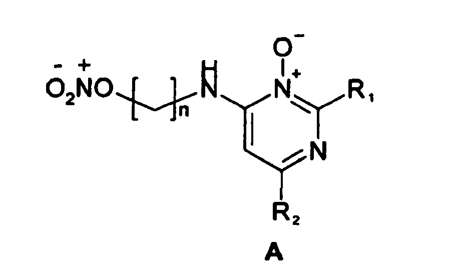 dans laquelle :
- n représente un entier allant de 2 à 12,
- R₁ représente un radical alkyle, linéaire ou ramifié, saturé ou insaturé, éventuellement substitué par un groupe -OR', -NR'R" ou -COOR', R₁ ayant de 1 à 20 atomes de carbone et mieux de 1 à 10 atomes de carbone, ou représente NR'R"
- R₂ représente l'hydrogène -NR₃R₄, -OR₃, -SR₃, avec R₃ et R₄ représentant indépendamment un radical alkyle, linéaire ou ramifié, saturé ou insaturé, éventuellement substitué par un groupe -OR', -NR'R", -COOR', R₂ ayant de 1 à 20 atomes de carbone et mieux de 1 à 10 atomes de carbone, R₃ et R₄ pouvant également faire partie d'un cycle de 4 à 7 atomes, saturé ou insaturé, contenant éventuellement au moins un hétéroatome,
- avec R' et R" égaux ou différents représentant l'hydrogène ou un radical alkyle entre C₁-C₃, linéaire ou ramifié, saturé,
pour la fabrication d'une composition capillaire d'être humain, destinée à traiter les désordres liés à une réduction de la microcirculation d'un follicule pileux.

17. Utilisation selon l'une des revendications 12 à 16, **caractérisée en ce que** n est un entier allant de 2 à 6 et mieux de 2 à 4.

18. Utilisation selon l'une des revendications 12 à 17, **caractérisée en ce** l'un au moins de R₁ et R₂ représentent un hydrogène.

19. Utilisation selon l'une des revendications 12 à 18, **caractérisée en ce que** R₁ représente NRR'.

20. Utilisation selon la revendication précédente, **caractérisée en ce que** R et R' représentent l'hydrogène.

21. Utilisation selon l'une des revendications 12 à 20, **caractérisée en ce que** R₂ représente NR₃R₄.

22. Utilisation selon l'une des revendications 12 à 21, **caractérisée en ce que** R₃ et R₄ forment avec l'azote auquel ils sont liés un hétérocycle.

23. Utilisation selon l'une des revendications 12 à 22, **caractérisée en ce que** R₂ représente l'hydrogène et R₁ représente NRR' avec R et R' valant H ou **en ce que** R₂ représente un hétérocycle saturé à 6 atomes, l'hétéroatome étant l'azote et R₁ représente NRR' avec R et R' valant H.

24. Utilisation selon l'une des revendications 12 à 23, **caractérisée en ce que** le sel du composé de formule (A) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), les sels de tri-éthanolamine, mono-éthanolamine, di-éthanolamine, hexadécylamine, N,N,N',N'-tétrakis-(hydroxy-propyl-2) éthylène di-amine, tris-hydroxyméthylaminométhane, les hydroxydes, les carbonates, les chlorures, les sulfates, les citrates, les acétates, les lactates.

25. Utilisation selon l'une des revendications 12 à 24, **caractérisée en ce que** le dérivé satisfait à l'une des formules suivantes :

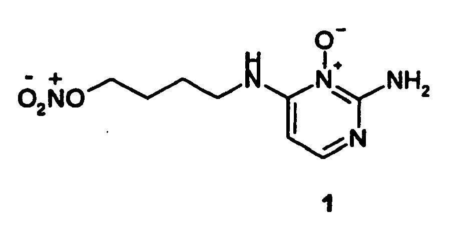

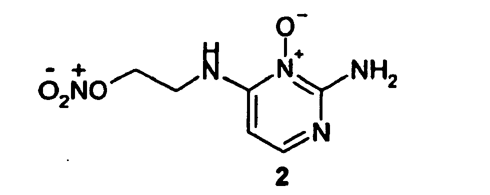

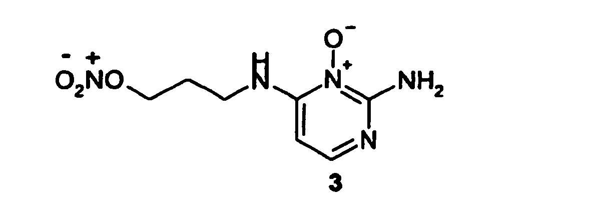

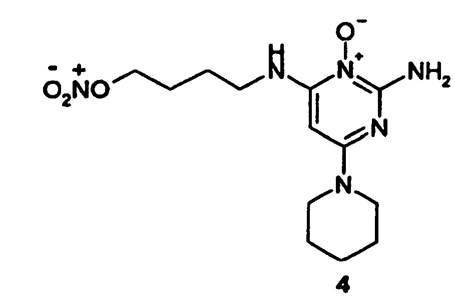

26. Utilisation selon l'une des revendications 12 à 25, **caractérisée en ce que** le dérivé est utilisé à une concentration allant de 10⁻³ à 5%, de préférence de 10⁻² à 2%, par rapport au poids total de la composition.

27. Utilisation selon l'une des revendications 12 à 26, **caractérisée en ce que** la composition est une composition à application topique.

28. Composition contenant un milieu physiologiquement acceptable et une quantité efficace d'au moins un dérivé de formule (A) ou de l'un de ses sels, conforme à l'une des revendications 1 à 11.

29. Composition selon la revendication 28, **caractérisée en ce que** la composition est une composition à application topique.

30. Composition selon la revendication 28 ou 29, **caractérisée en ce qu'**elle se présente sous forme de crème ou lotion capillaire, de shampooing ou d'après-shampooing capillaire, de mascara pour les cils ou les cheveux.

31. Composition selon l'une des revendications 28 à 30, **caractérisée en ce qu'**elle est sous forme de solution ou suspension aqueuse, alcoolique ou hydro-alcoolique.

32. Composition selon l'une des revendications 28 à 31, **caractérisée en ce qu'**elle contient d'autres ingrédients choisis parmi les solvants, les épaississants ou gélifiants de phase aqueuse ou de phase huileuse, les matières colorantes solubles dans le milieu de la composition, les charges, les pigments, les antioxydants, les conservateurs, les parfums, les électrolytes, les neutralisants, les polymères filmogènes, les agents bloqueurs d'U.V., les actifs cosmétiques et pharmaceutiques à action bénéfique pour la peau ou les fibres kératiniques, leurs mélanges.

33. Composition selon l'une des revendications 28 à 32, **caractérisée en ce qu'**elle contient au moins un composé additionnel favorisant la repousse et/ou limitant la chute des cheveux, choisi parmi l'aminexil, le 6-0-[(9Z,12Z)-octadéca-9,12-diènoyl]hexapyranose, les inhibiteurs de lipoxygénase, les inhibiteurs de bradykinine, les prostaglandines et leurs dérivés, les agonistes ou antagonistes des récepteurs des prostaglandines, les analogues non prostanoïques de prostaglandines, les vasodilatateurs, les antiandrogènes, les cyclosporines et leurs analogues, les antimicrobiens, les anti-inflammatoires, les rétinoïdes, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les vitamines, les hydroxy-acides, les benzophénones, l'hydantoïne, l'octopirox, l'acide rétinoïque, les agents antiprurigineux, les antiparasitaires, les antifongiques, les esters d'acide nicotinique, les agents antagonistes de calcium, les hormones, les triterpènes, les agents antiandrogènes, les inhibiteurs stéroïdiens ou non stéroïdiens des 5-α-réductases, les agonistes de canaux potassiques, les agonistes du récepteur FP, les inhibiteurs de 15 hydroxyprostaglandine déshydrogénase, leurs mélanges.

34. Composition selon l'une des revendications 28 à 33, **caractérisée en ce qu'**elle contient, en outre, un autre actif additionnel choisi parmi les protéines, les hydrolysats de protéine, les acides aminés, les polyols, l'urée, l'allantoïne, les sucres et les dérivés de sucre, les extraits végétaux (ceux d'Iridacées ou de soja), des hydroxy-acides ; les dérivés du rétinol ou du tocophérol, les acides gras essentiels, les céramides, les huiles essentielles, les dérivés de l'acide salicylique, les esters des hydroxy-acides, les phospholipides, leurs mélanges.

35. Procédé de fabrication d'un dérivé de pyrimidine N-oxyde conforme à l'une des revendications 1 à 11, comprenant les étapes suivantes :
1°) Réaction du composé **5** 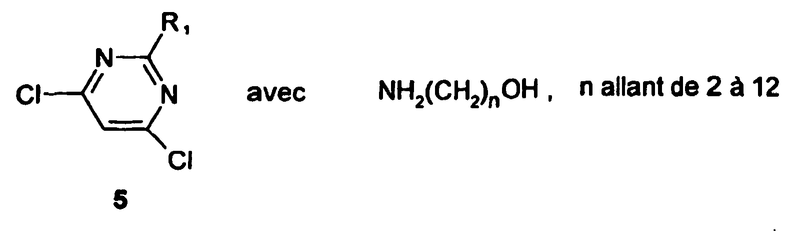
2°) Oxydation du produit obtenu à l'étape 1°),
3°) Substitution du produit obtenu à l'étape 2°) pour substituer un atome de chlore par un substituant,
4°) Nitration du produit obtenu à l'étape 3°).

## Claims

1. Pyrimidine N-oxide derivative of formula (A) or one of its salts: 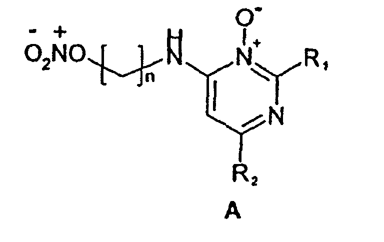 in which:
- n represents an integer ranging from 2 to 12,
- R₁ represents a saturated or unsaturated, linear or branched alkyl radical optionally substituted with an
- OR', -NR'R" or -COOR' group, R₁ having from 1 to 20 carbon atoms, and better still from 1 to 10 carbon atoms, or represents NR'R",
- R₂ represents hydrogen, -NR₃R₄, -OR₃ or -SR₃, with R₃ and R₄ independently representing a saturated or unsaturated, linear or branched alkyl radical optionally substituted with an -OR', -NR'R" or -COOR' group, R₂ having from 1 to 20 carbon atoms, and better still from 1 to 10 carbon atoms, it also being possible for R₃ and R₄ to be part of a saturated or unsaturated, 4- to 7-atom ring optionally containing at least one heteroatom,
- with R' and R", which may be equal or different, representing hydrogen or a saturated, linear or branched C₁-C₃ alkyl radical.

2. Derivative according to the preceding claim, **characterized in that** n is an integer ranging from 2 to 6, and better still from 2 to 4.

3. Derivative according to either of the preceding claims, **characterized in that** at least one of R₁ and R₂ represents a hydrogen.

4. Derivative according to one of the preceding claims, **characterized in that** R₁ represents NRR'.

5. Derivative according to the preceding claim, **characterized in that** R and R' represent hydrogen.

6. Derivative according to one of the preceding claims, **characterized in that** R₂ represents NR₃R₄.

7. Derivative according to one of the preceding claims, **characterized in that** R₃ and R₄ are part of a heterocycle.

8. Derivative according to one of the preceding claims, **characterized in that** R₃ and R₄ form, with the nitrogen to which they are attached, a heterocycle.

9. Derivative according to one of the preceding claims, **characterized in that** R₂ represents hydrogen and R₁ represents NRR', with R and R' being H, or **in that** R₂ represents a 6-atom saturated heterocycle, the heteroatom being nitrogen, and R₁ represents NRR' with R and R' being H.

10. Derivative according to one of the preceding claims, **characterized in that** the salt of the derivative of formula (A) is a salt chosen from sodium salts, potassium salts, zinc (Zn²⁺) salts, calcium (Ca²⁺) salts, copper (Cu²⁺) salts, iron (Fe²⁺) salts, strontium (Sr²⁺) salts, magnesium (Mg²⁺) salts, manganese (Mn²⁺) salts, triethanolamine salts, monoethanolamine salts, diethanolamine salts, hexadecylamine salts, N,N,N',N'-tetrakis(2-hydroxypropyl)ethylenediamine salts, trishydroxymethylaminomethane salts, hydroxides, carbonates, chlorides, sulphates, citrates, acetates and lactates.

11. Derivative according to one of the preceding claims, **characterized in that** the derivative corresponds to one of the following formulae:

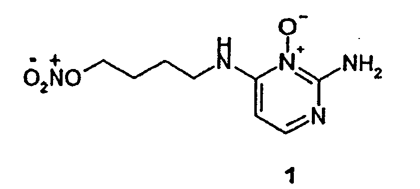

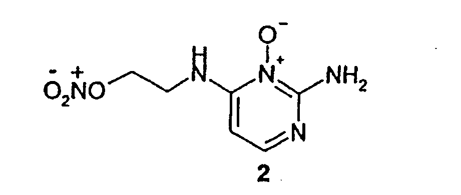

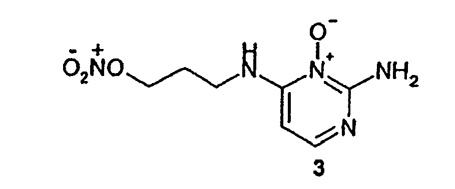

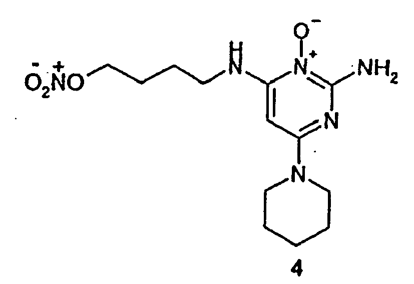

12. Use of at least one pyrimidine N-oxide derivative of formula (A) or of one of its salts 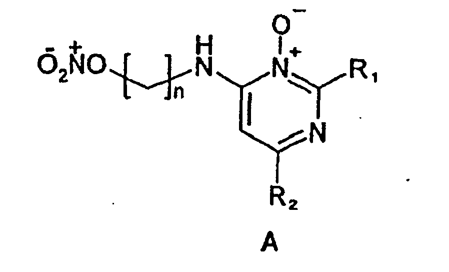 in which:
- n represents an integer ranging from 2 to 12,
- R₁ represents a saturated or unsaturated, linear or branched alkyl radical optionally substituted with an -OR', -NR'R" or -COOR' group, R₁ having from 1 to 20 carbon atoms, and better still from 1 to 10 carbon atoms, or represents NR'R",
- R₂ represents hydrogen, -NR₃R₄, -OR₃ or -SR₃, with R₃ and R₄ independently representing a saturated or unsaturated, linear or branched alkyl radical optionally substituted with an -OR', -NR'R" or -COOR' group, R₂ having from 1 to 20 carbon atoms, and better still from 1 to 10 carbon atoms, it also being possible for R₃ and R₄ to be part of a saturated or unsaturated, 4- to 7-atom ring optionally containing at least one heteroatom,
- with R' and R", which may be equal or different, representing hydrogen or a saturated, linear or branched C₁-C₃ alkyl radical,
in the manufacture of a composition for use in treating disorders related to the synthesis and/or release of lysyl hydroxylase and/or related to a reduction in the microcirculation of the skin.

13. Use of at least one pyrimidine N-oxide derivative of formula (A) or of one of its salts 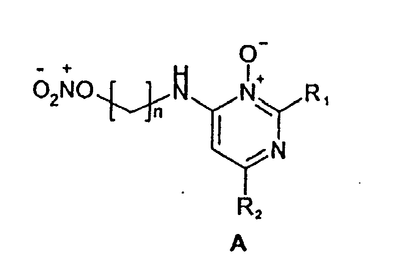 in which:
- n represents an integer ranging from 2 to 12,
- R₁ represents a saturated or unsaturated, linear or branched alkyl radical optionally substituted with an -OR', -NR'R" or -COOR' group, R₁ having from 1 to 20 carbon atoms, and better still from 1 to 10 carbon atoms, or represents NR'R",
- R₂ represents hydrogen, -NR₃R₄, -OR₃ or -SR₃, with R₃ and R₄ independently representing a saturated or unsaturated, linear or branched alkyl radical optionally substituted with an -OR', -NR'R" or -COOR' group, R₂ having from 1 to 20 carbon atoms, and better still from 1 to 10 carbon atoms, it also being possible for R₃ and R₄ to be part of a saturated or unsaturated, 4- to 7-atom ring optionally containing at least one heteroatom,
- with R' and R", which may be equal or different, representing hydrogen or a saturated, linear or branched C₁-C₃ alkyl radical,
in the preparation of a composition for caring for and/or for treating human keratin fibres, intended to induce and/or stimulate the growth of human keratin fibres and/or to stop the loss thereof and/or to increase the density thereof.

14. Use of at least one pyrimidine N-oxide derivative of formula (A) or of one of its salts 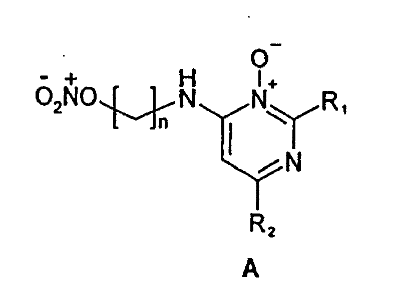 in which:
- n represents an integer ranging from 2 to 12,
- R₁ represents a saturated or unsaturated, linear or branched alkyl radical optionally substituted with an -OR', -NR'R" or -COOR' group, R₁ having from 1 to 20 carbon atoms, and better still from 1 to 10 carbon atoms, or represents -NR'R",
- R₂ represents hydrogen, -NR₃R₄, -OR₃ or -SR₃, with R₃ and R₄ independently representing a saturated or unsaturated, linear or branched alkyl radical optionally substituted with an -OR', -NR'R" or -COOR' group, R₂ having from 1 to 20 carbon atoms, and better still from 1 to 10 carbon atoms, it also being possible for R₃ and R₄ to form, together, a saturated or unsaturated, 4- to 7-atom ring optionally containing at least one heteroatom,
- with R' and R", which may be equal or different, representing hydrogen or a saturated, linear or branched C₁-C₃ alkyl radical,
in the preparation of a physiologically acceptable composition, for use in treating the intrinsic and extrinsic signs of ageing, excess weight, ulcers, Raynaud's syndrome, psoriasis, skin mycoses, inflammatory disorders, contact hypersensitivity reactions, allergic manifestations, eczema, pruritus, sensitive skin, hypermelanosis, and/or improving skin cicatrization.

15. Use of at least one pyrimidine N-oxide derivative of formula (A) or of one of its salts 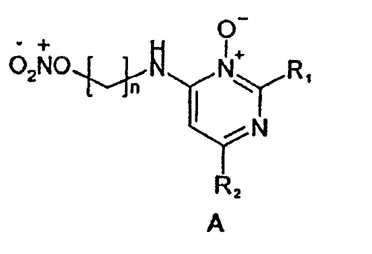 in which:
- n represents an integer ranging from 2 to 12,
- R₁ represents a saturated or unsaturated, linear or branched alkyl radical optionally substituted with an -OR', -NR'R" or -COOR' group, R₁ having from 1 to 20 carbon atoms, and better still from 1 to 10 carbon atoms, or represents NR'R",
- R₂ represents hydrogen, -NR₃R₄, -OR₃ or -SR₃, with R₃ and R₄ independently representing a saturated or unsaturated, linear or branched alkyl radical optionally substituted with an -OR', -NR'R" or -COOR' group, R₂ having from 1 to 20 carbon atoms, and better still from 1 to 10 carbon atoms, it also being possible for R₃ and R₄ to be part of a saturated or unsaturated, 4- to 7-atom ring optionally containing at least one heteroatom,
- with R' and R", which may be equal or different, representing hydrogen or a saturated, linear or branched C₁-C₃ alkyl radical,
in the manufacture of a hair composition for human beings, for use in inducing and/or stimulating the growth of the hair and/or stopping the loss thereof and/or increasing the density thereof and/or treating androgenic alopecia.

16. Use of at least one pyrimidine N-oxide derivative of formula (A) or of one of its salts 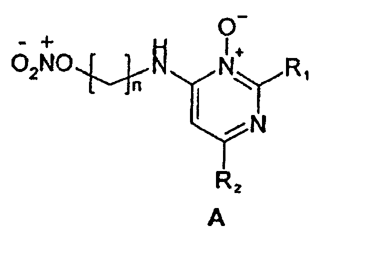 in which:
- n represents an integer ranging from 2 to 12,
- R₁ represents a saturated or unsaturated, linear or branched alkyl radical optionally substituted with an -OR', -NR'R" or -COOR' group, R₁ having from 1 to 20 carbon atoms, and better still from 1 to 10 carbon atoms, or represents NR'R",
- R₂ represents hydrogen, -NR₃R₄, -OR₃ or -SR₃, with R₃ and R₄ independently representing a saturated or unsaturated, linear or branched alkyl radical optionally substituted with an -OR', -NR'R" or -COOR' group, R₂ having from 1 to 20 carbon atoms, and better still from 1 to 10 carbon atoms, it also being possible for R₃ and R₄ to be part of a saturated or unsaturated, 4- to 7-atom ring optionally containing at least one heteroatom,
- with R' and R", which may be equal or different, representing hydrogen or a saturated, linear or branched C₁-C₃ alkyl radical,
in the manufacture of a hair composition for human beings, for use in treating disorders related to a reduction in the microcirculation of a hair follicle.

17. Use according to one of Claims 12 to 16, **characterized in that** n is an integer ranging from 2 to 6, and better still from 2 to 4.

18. Use according to one of Claims 12 to 17, **characterized in that** at least one of R₁ and R₂ represents a hydrogen.

19. Use according to one of Claims 12 to 18, **characterized in that** R₁ represents NRR'.

20. Use according to the preceding claim, **characterized in that** R and R' represent hydrogen.

21. Use according to one of Claims 12 to 20, **characterized in that** R₂ represents NR₃R₄.

22. Use according to one of Claims 12 to 21, **characterized in that** R₃ and R₄ form, with the nitrogen to which they are attached, a heterocycle.

23. Use according to one of Claims 12 to 22, **characterized in that** R₂ represents hydrogen and R₁ represents NRR' with R and R' being H, or **in that** R₂ represents a 6-atom saturated heterocycle, the heteroatom being nitrogen, and R₁ represents NRR' with R and R' being H.

24. Use according to one of Claims 12 to 23, **characterized in that** the salt of the compound of formula (A) is a salt chosen from sodium salts, potassium salts, zinc (Zn²⁺) salts, calcium (Ca²⁺) salts, copper (Cu²⁺) salts, iron (Fe²⁺) salts, strontium (Sr²⁺) salts, magnesium (Mg²⁺) salts, manganese (Mn²⁺) salts, triethanolamine salts, monoethanolamine salts, diethanolamine salts, hexadecylamine salts, N,N,N',N'-tetrakis(2-hydroxypropyl)ethylenediamine salts, trishydroxymethylaminomethane salts, hydroxides, carbonates, chlorides, sulphates, citrates, acetates and lactates.

25. Use according to one of Claims 12 to 24, **characterized in that** the derivative corresponds to one of the following formulae:

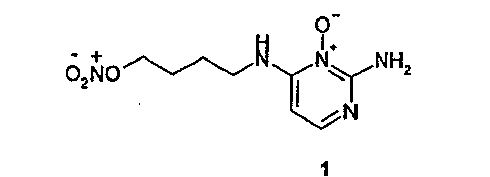

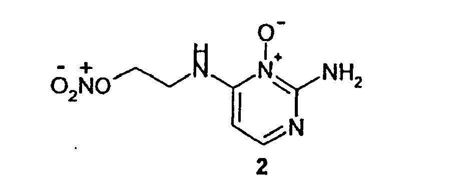

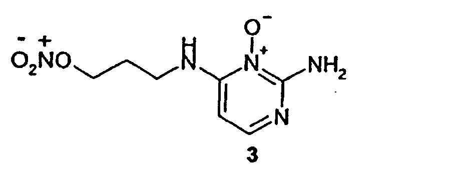

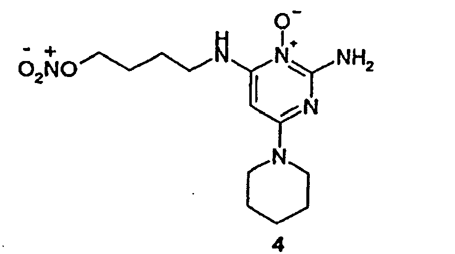

26. Use according to one of Claims 12 to 25, **characterized in that** the derivative is used at a concentration ranging from 10⁻³% to 5%, preferably from 10⁻²% to 2%, relative to the total weight of the composition.

27. Use according to one of Claims 12 to 26, **characterized in that** the composition is a composition for topical application.

28. Composition containing a physiologically acceptable medium and an effective amount of at least one derivative of formula (A) or of one of its salts, in accordance with one of Claims 1 to 11.

29. Composition according to Claim 28, **characterized in that** the composition is a composition for topical application.

30. Composition according to Claim 28 or 29, **characterized in that** it is in the form of a hair cream or hair lotion, of a shampoo or a conditioner for the hair, or of a mascara for the eyelashes or the hair.

31. Composition according to one of Claims 28 to 30, **characterized in that** it is in the form of an aqueous, alcoholic or aqueous-alcoholic solution or suspension.

32. Composition according to one of Claims 28 to 31, **characterized in that** it contains other ingredients chosen from solvents, aqueous-phase or oily-phase thickeners or gelling agents, dyestuffs that are soluble in the medium of the composition, fillers, pigments, antioxidants, preserving agents, fragrances, electrolytes, neutralizers, film-forming polymers, UV blockers, cosmetic and pharmaceutical active agents with a beneficial effect on the skin or keratin fibres, and mixtures thereof.

33. Composition according to one of Claims 28 to 32, **characterized in that** it contains at least one additional compound that promotes hair regrowth and/or limits hair loss, chosen from aminexil, 6-0-[(9Z,12Z)-octadeca-9,12-dienoyl]hexapyranose, lipoxygenase inhibitors, bradykinin inhibitors, prostaglandins and derivatives thereof, prostaglandin receptor agonists or antagonists, non-prostanoic analogues of prostaglandins, vasodilators, antiandrogenic agents, cyclosporins and analogues thereof, antimicrobial agents, anti-inflammatories, retinoids, benzalkonium chloride, benzethonium chloride, phenol, oestradiol, chloropheniramine maleate, chlorophylline derivatives, cholesterol, cysteine, methionine, menthol, peppermint oil, calcium pantothenate, panthenol, resorcinol, protein kinase C activators, glycosidase inhibitors, glycosaminoglycanase inhibitors, pyroglutamic acid esters, hexosaccharide or acylhexosaccharide acids, substituted arylethylenes, N-acylamino acids, flavonoids, ascomycin derivatives and analogues, histamine antagonists, saponins, proteoglycanase inhibitors, oestrogen agonists and antagonists, pseudoterines, cytokines, growth factor promoters, inhibitors of IL-1 or of IL-6, IL-10 promoters, TNF inhibitors, vitamins, hydroxy acids, benzophenones, hydantoin, octopirox, retinoic acid, antipruriginous agents, antiparasitic agents, antifungal agents, nicotinic acid esters, calcium antagonists, hormones, triterpenes, anti-androgenic agents, steroidal or non-steroidal inhibitors of 5-α-reductases, potassium channel agonists, FP receptor agonists, 15-hydroxy-prostaglandin dehydrogenase inhibitors, and mixtures thereof.

34. Composition according to one of Claims 28 to 33, **characterized in that** it also contains another additional active agent chosen from proteins, protein hydrolysates, amino acids, polyols, urea, allantoin, sugars and sugar derivatives, plant extracts (those of Iridacaea or of soybean), hydroxy acids; derivatives of retinol or of tocopherol, essential fatty acids, ceramides, essential oils, salicylic acid derivatives, hydroxy acid esters, phospholipids, and mixtures thereof.

35. Process for the manufacture of a pyrimidine N-oxide derivative in accordance with one of Claims 1 to 11, comprising the following steps:
1°) reaction of compound **5** 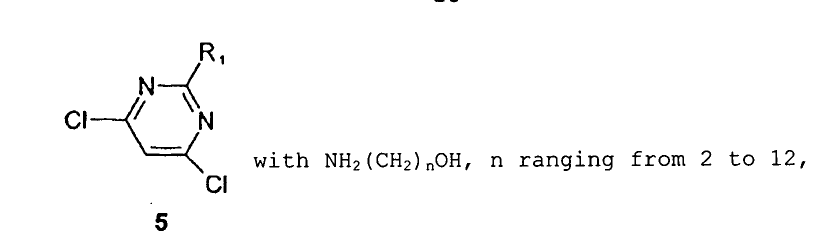
2°) oxidation of the product obtained in step 1°),
3°) substitution of the product obtained in step 2°) so as to substitute a chlorine atom with a substituent,
4°) nitration of the product obtained in step 3°).

## Patentansprüche

1. Pyrimidin-*N*-oxid-Derivat der Formel (A) 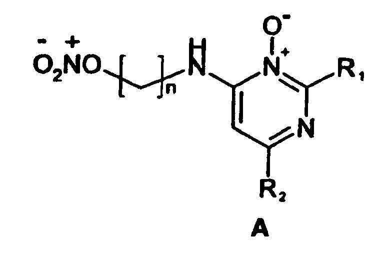 oder eines seiner Salze, in der:
- n eine ganze Zahl darstellt, die im Bereich von 2 bis 12 liegt,
- R₁ einen geradkettigen oder verzweigten, gesättigten oder ungesättigten Alkylrest, der gegebenenfalls mit einer Gruppe -OR', -NR'R" oder -COOR' substituiert ist, wobei R₁ 1 bis 20 Kohlenstoffatome und besser 1 bis 10 Kohlenstoffatome aufweist, oder einen Rest -NR'R" darstellt,
- R₂ Wasserstoff, -NR₃R₄, -OR₃, -SR₃ bedeutet, wobei R₃ und R₄ unabhängig einen geradkettigen oder verzweigten, gesättigten oder ungesättigten Alkylrest darstellen, der gegebenenfalls mit einer Gruppe -OR', -NR'R", -COOR' substituiert ist, wobei R₂ 1 bis 20 Kohlenstoffatome und besser 1 bis 10 Kohlenstoffatome aufweist, wobei R₃ und R₄ gegebenenfalls Teil eines gesättigten oder ungesättigten Rings aus 4 bis 7 Atomen sein können, der gegebenenfalls mindestens ein Heteroatom enthält,
- wobei R' und R", die gleich oder verschieden sind, Wasserstoff oder einen geradkettigen oder verzweigten, gesättigten C₁₋₃-Alkylrest bedeuten.

2. Derivat nach dem vorhergehenden Anspruch, **dadurch gekennzeichnet, dass** n eine ganze Zahl ist, die im Bereich von 2 bis 6 und besser 2 bis 4 liegt.

3. Derivat nach einem der vorhergehenden Ansprüche, **dadurch gekennzeichnet, dass** mindestens einer der Reste R₁ und R₂ Wasserstoff bedeutet.

4. Derivat nach einem der vorhergehenden Ansprüche, **dadurch gekennzeichnet, dass** R₁ -NRR' bedeutet.

5. Derivat nach dem vorhergehenden Anspruch, **dadurch gekennzeichnet, dass** R und R' Wasserstoff bedeuten.

6. Derivat nach einem der vorhergehenden Ansprüche, **dadurch gekennzeichnet, dass** R₂ -NR₃R₄ bedeutet.

7. Derivat nach einem der vorhergehenden Ansprüche, **dadurch gekennzeichnet, dass** R₃ und R₄ Teil eines Heterocyclus sind.

8. Derivat nach einem der vorhergehenden Ansprüche, **dadurch gekennzeichnet, dass** R₃ und R₄ mit dem Stickstoffatom, mit dem sie verbunden sind, einen Heterocyclus bilden.

9. Derivat nach einem der vorhergehenden Ansprüche, **dadurch gekennzeichnet, dass** R₂ Wasserstoff bedeutet und R₁ -NRR' bedeutet, worin R und R' Wasserstoffatome sind, oder dass R₂ einen gesättigten Heterocyclus aus 6 Atomen darstellt, wobei Stickstoff das Heteroatom ist, und R₁ -NRR' bedeutet, worin R und R' Wasserstoffatome sind.

10. Derivat nach einem der vorhergehenden Ansprüche, **dadurch gekennzeichnet, dass** das Salz des Derivats der Formel (A) ein Salz ist, das unter den Salzen von Natrium, Kalium, den Salzen von Zink (Zn²⁺), Calcium (Ca²⁺), Kupfer (Cu²⁺), Eisen (Fe²⁺), Strontium (Sr²⁺), Magnesium (Mg²⁺), Mangan (Mn²⁺), den Salzen von Triethanolamin, Monoethanolamin, Diethanolamin, Hexadecylamin, N,N,N',N'-Tetrakis-(2-hydroxypropyl)-ethylendiamin, Trishydroxymethylaminomethan, den Hydroxiden, den Carbonaten und den Chloriden, Sulfaten, Citraten, Acetaten, Lactaten ausgewählt wird.

11. Derivat nach einem der vorhergehenden Ansprüche, **dadurch gekennzeichnet, dass** das Derivat einer der folgenden Formeln genügt:

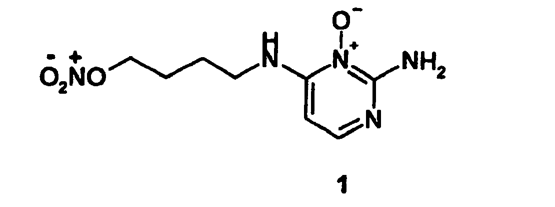

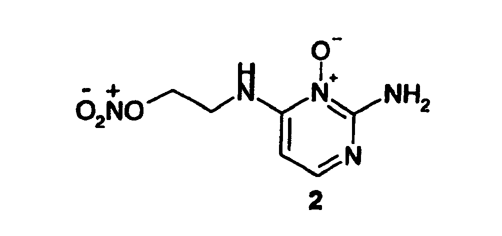

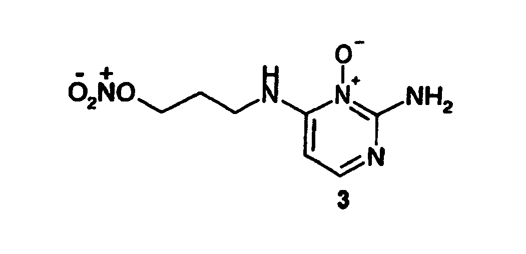

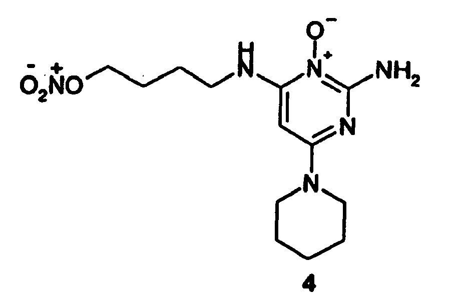

12. Verwendung mindestens eines Pyrimidin-*N*-oxid-Derivats der Formel (A) 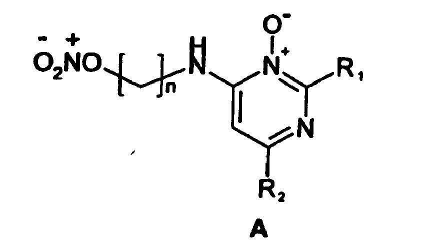 oder eines seiner Salze, in der:
- n eine ganze Zahl darstellt, die im Bereich von 2 bis 12 liegt,
- R₁ einen geradkettigen oder verzweigten, gesättigten oder ungesättigten Alkylrest, der gegebenenfalls mit einer Gruppe -OR', -NR'R" oder -COOR' substituiert ist, wobei R₁ 1 bis 20 Kohlenstoffatome und besser 1 bis 10 Kohlenstoffatome aufweist, oder einen Rest -NR'R" darstellt,
- R₂ Wasserstoff, -NR₃R₄, -OR₃, -SR₃ bedeutet, wobei R₃ und R₄ unabhängig einen geradkettigen oder verzweigten, gesättigten oder ungesättigten Alkylrest darstellen, der gegebenenfalls mit einer Gruppe -OR', -NR'R", -COOR' substituiert ist, wobei R₂ 1 bis 20 Kohlenstoffatome und besser 1 bis 10 Kohlenstoffatome aufweist, wobei R₃ und R₄ gegebenenfalls Teil eines gesättigten oder ungesättigten Rings aus 4 bis 7 Atomen sein können, der gegebenenfalls mindestens ein Heteroatom enthält,
- wobei R' und R", die gleich oder verschieden sind, Wasserstoff oder einen geradkettigen oder verzweigten, gesättigten C₁₋₃-Alkylrest bedeuten.
für die Herstellung einer Zusammensetzung, die für die Behandlung der Störungen vorgesehen ist, die mit der Synthese und/oder Freisetzung der Lysyl-Hydroxylase zusammenhängen und/ oder die mit der Verringerung der Mikrozirkulation in der Haut zusammenhängen.

13. Verwendung mindestens eines Pyrimidin-*N*-oxid-Derivats der Formel (A) 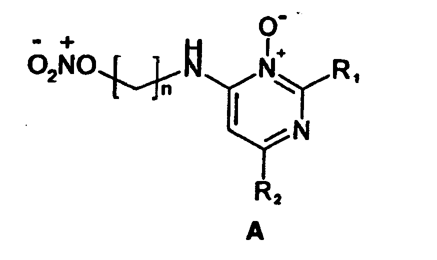 oder eines seiner Salze, in der:
- n eine ganze Zahl darstellt, die im Bereich von 2 bis 12 liegt,
- R₁ einen geradkettigen oder verzweigten, gesättigten oder ungesättigten Alkylrest, der gegebenenfalls mit einer Gruppe -OR', -NR'R" oder -COOR' substituiert ist, wobei R₁ 1 bis 20 Kohlenstoffatome und besser 1 bis 10 Kohlenstoffatome aufweist, oder einen Rest -NR'R" darstellt,
- R₂ Wasserstoff, -NR₃R₄, -OR₃, -SR₃ bedeutet, wobei R₃ und R₄ unabhängig einen geradkettigen oder verzweigten, gesättigten oder ungesättigten Alkylrest darstellen, der gegebenenfalls mit einer Gruppe -OR', -NR'R", -COOR' substituiert ist, wobei R₂ 1 bis 20 Kohlenstoffatome und besser 1 bis 10 Kohlenstoffatome aufweist, wobei R₃ und R₄ gegebenenfalls Teil eines gesättigten oder ungesättigten Rings aus 4 bis 7 Atomen sein können, der gegebenenfalls mindestens ein Heteroatom enthält,
- wobei R' und R", die gleich oder verschieden sind, Wasserstoff oder einen geradkettigen oder verzweigten, gesättigten C₁₋₃-Alkylrest bedeuten
für die Herstellung einer Zusammensetzung zum Pflegen und/oder Behandeln von menschlichen Keratinfasern, die dafür vorgesehen ist, das Wachstum menschlicher Keratinfasern auszulösen und/oder zu stimulieren und/oder ihren Ausfall zu bremsen und/oder ihre Dichte zu erhöhen.

14. Verwendung mindestens eines Pyrimidin-*N*-oxid-Derivats der Formel (A) 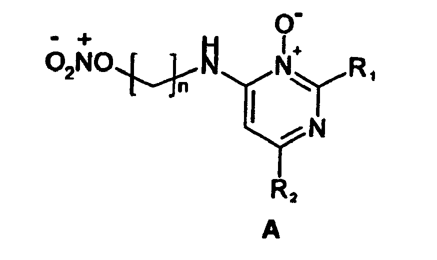 oder eines seiner Salze, in der:
- n eine ganze Zahl darstellt, die im Bereich von 2 bis 12 liegt,
- R₁ einen geradkettigen oder verzweigten, gesättigten oder ungesättigten Alkylrest, der gegebenenfalls mit einer Gruppe -OR', -NR'R" oder -COOR' substituiert ist, wobei R₁ bis 20 Kohlenstoffatome und besser 1 bis 10 Kohlenstoffatome aufweist, oder einen Rest -NR'R" darstellt,
- R₂ Wasserstoff, -NR₃R₄, -OR₃, -SR₃ bedeutet, wobei R₃ und R₄ unabhängig einen geradkettigen oder verzweigten, gesättigten oder ungesättigten Alkylrest darstellen, der gegebenenfalls mit einer Gruppe -OR', -NR'R", -COOR' substituiert ist, wobei R₂ 1 bis 20 Kohlenstoffatome und besser 1 bis 10 Kohlenstoffatome aufweist, wobei R₃ und R₄ außerdem miteinander einen gesättigten oder ungesättigten Ring aus 4 bis 7 Atomen bilden können, der gegebenenfalls mindestens ein Heteroatom enthält,
- wobei R' und R", die gleich oder verschieden sind, Wasserstoff oder einen geradkettigen oder verzweigten, gesättigten C₁₋₃-Alkylrest bedeuten
für die Herstellung einer physiologisch akzeptablen Zusammensetzung, die für die Behandlung der Anzeichen der inneren Alterung und der durch äußere Einflüsse hervorgerufenen Alterung, des Übergewichts, von Geschwüren, des Raynaud-Syndroms, der Psoriasis, der Mykosen der Haut, der Entzündungskrankheiten, der Kontakthypersensibilitätsreaktionen, des Zutagetretens von allergischen Reaktionen, der Exzeme, von Juckreiz, von sensibler Haut, der Hypermelanose und/oder für die Verbesserung der Heilung der Haut vorgesehen ist.

15. Verwendung mindestens eines Pyrimidin-*N*-oxid-Derivats der Formel (A) 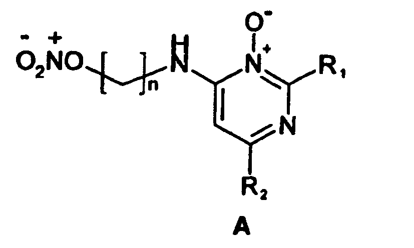 oder eines seiner Salze, in der:
- n eine ganze Zahl darstellt, die im Bereich von 2 bis 12 liegt,
- R₁ einen geradkettigen oder verzweigten, gesättigten oder ungesättigten Alkylrest, der gegebenenfalls mit einer Gruppe -OR', -NR'R" oder -COOR' substituiert ist, wobei R₁ 1 bis 20 Kohlenstoffatome und besser 1 bis 10 Kohlenstoffatome aufweist, oder einen Rest -NR'R" darstellt,
- R₂ Wasserstoff, -NR₃R₄, -OR₃, -SR₃ bedeutet, wobei R₃ und R₄ unabhängig einen geradkettigen oder verzweigten, gesättigten oder ungesättigten Alkylrest darstellen, der gegebenenfalls mit einer Gruppe -OR', -NR'R", -COOR' substituiert ist, wobei R₂ 1 bis 20 Kohlenstoffatome und besser 1 bis 10 Kohlenstoffatome aufweist, wobei R₃ und R₄ gegebenenfalls Teil eines gesättigten oder ungesättigten Rings aus 4 bis 7 Atomen sein können, der gegebenenfalls mindestens ein Heteroatom enthält,
- wobei R' und R", die gleich oder verschieden sind, Wasserstoff oder einen geradkettigen oder verzweigten, gesättigten C₁₋₃-Alkylrest bedeuten
für die Herstellung einer Zusammensetzung zur Behandlung der Haare des Menschen, die dafür vorgesehen ist, das Wachstum der Haare hervorzurufen und/oder zu stimulieren und/oder ihren Ausfall zu bremsen und/oder ihre Dichte zu vergrößern und oder den androgenen Haarausfall zu behandeln.

16. Verwendung mindestens eines Pyrimidin-*N*-oxid-Derivats der Formel (A) 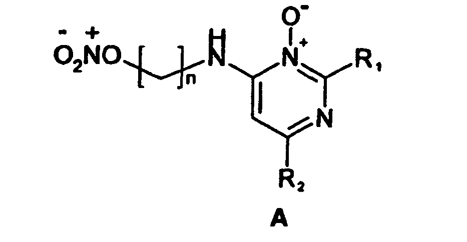 oder eines seiner Salze, in der:
- n eine ganze Zahl darstellt, die im Bereich von 2 bis 12 liegt,
- R₁ einen geradkettigen oder verzweigten, gesättigten oder ungesättigten Alkylrest, der gegebenenfalls mit einer Gruppe -OR', -NR'R" oder -COOR' substituiert ist, wobei R₁ 1 bis 20 Kohlenstoffatome und besser 1 bis 10 Kohlenstoffatome aufweist, oder einen Rest -NR'R" darstellt,
- R₂ Wasserstoff, -NR₃R₄, -OR₃, -SR₃ bedeutet, wobei R₃ und R₄ unabhängig einen geradkettigen oder verzweigten, gesättigten oder ungesättigten Alkylrest darstellen, der gegebenenfalls mit einer Gruppe -OR', -NR'R", -COOR' substituiert ist, wobei R₂ 1 bis 20 Kohlenstoffatome und besser 1 bis 10 Kohlenstoffatome aufweist, wobei R₃ und R₄ gegebenenfalls Teil eines gesättigten oder ungesättigten Rings aus 4 bis 7 Atomen sein können, der gegebenenfalls mindestens ein Heteroatom enthält,
- wobei R' und R", die gleich oder verschieden sind, Wasserstoff oder einen geradkettigen oder verzweigten, gesättigten C₁₋₃-Alkylrest bedeuten,
für die Herstellung einer Zusammensetzung zur Behandlung der Haare des Menschen, die dafür vorgesehen ist, die Störungen zu behandeln, die mit einer Verringerung der Mikrozirkulation der Haarfollikel zusammenhängen.

17. Verwendung nach einem der Ansprüche 12 bis 16, **dadurch gekennzeichnet, dass** n eine ganze Zahl ist, die im Bereich von 2 bis 6 und besser 2 bis 4 liegt.

18. Verwendung nach einem der Ansprüche 12 bis 17, **dadurch gekennzeichnet, dass** mindestens einer der Reste R₁ und R₂ Wasserstoff bedeutet.

19. Verwendung nach einem der Ansprüche 12 bis 18, **dadurch gekennzeichnet, dass** R₁ -NRR' bedeutet.

20. Verwendung nach dem vorhergehenden Anspruch, **dadurch gekennzeichnet, dass** R und R' Wasserstoff bedeuten.

21. Verwendung nach einem der Ansprüche 12 bis 20, **dadurch gekennzeichnet, dass** R₂ -NR₃R₄ bedeutet.

22. Verwendung nach einem der Ansprüche 12 bis 21, **dadurch gekennzeichnet, dass** R₃ und R₄ mit dem Stickstoffatom, mit dem sie verbunden sind, einen Heterocyclus bilden.

23. Verwendung nach einem der Ansprüche 12 bis 22, **dadurch gekennzeichnet, dass** R₂ Wasserstoff bedeutet und R₁ -NRR' bedeutet , worin R und R' Wasserstoffatome sind, oder dass R₂ einen gesättigten Heterocyclus aus 6 Atomen darstellt, wobei Stickstoff das Heteroatom ist, und R₁ -NRR' bedeutet, worin R und R' Wasserstoffatome sind.

24. Verwendung nach einem der Ansprüche 12 bis 23, **dadurch gekennzeichnet, dass** das Salz der Verbindung der Formel (A) ein Salz ist, das unter den Salzen von Natrium, Kalium, den Salzen von Zink (Zn²⁺), Calcium (Ca²⁺), Kupfer (Cu²⁺), Eisen (Fe²⁺), Strontium (Sr²⁺), Magnesium (Mg²⁺), Mangan (Mn²⁺), den Salzen von Triethanolamin, Monoethanolamin, Diethanolamin, Hexadecylamin, N,N,N',N'-Tetrakis-(2-hydroxypropyl)-ethylendiamin, Trishydroxymethylaminomethan, den Hydroxiden, Carbonaten, Chloriden, Sulfaten, Citraten, Acetaten, Lactaten ausgewählt wird.

25. Verwendung nach einem der Ansprüche 12 bis 24, **dadurch gekennzeichnet, dass** das Derivat einer der folgenden Formeln genügt:

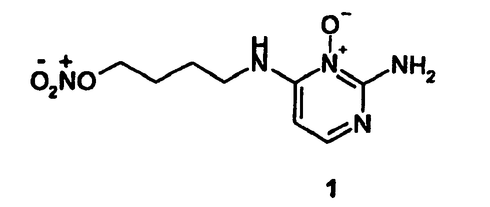

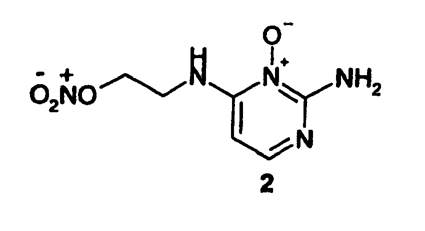

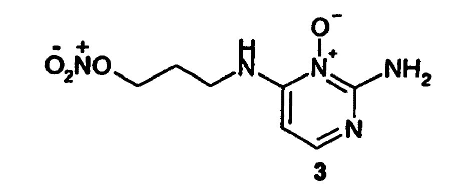

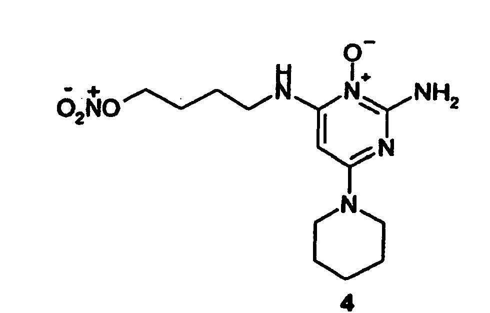

26. Verwendung nach einem der Ansprüche 12 bis 25, **dadurch gekennzeichnet, dass** das Derivat in einer Konzentration verwendet wird, die im Bereich von 10⁻³ bis 5 %, vorzugsweise 10⁻² bis 2 %, bezogen auf das Gesamtgewicht der Zusammensetzung, liegt.

27. Verwendung nach einem der Ansprüche 12 bis 26, **dadurch gekennzeichnet, dass** die Zusammensetzung eine Zusammensetzung für die topische Anwendung ist.

28. Zusammensetzung, die ein physiologisch akzeptables Medium und eine wirksame Menge mindestens eines Derivats der Formel (A) oder eines seiner Salze gemäß einem der Ansprüche 1 bis 11 enthält.

29. Zusammensetzung nach Anspruch 28, **dadurch gekennzeichnet, dass** die Zusammensetzung eine Zusammensetzung für die topische Anwendung ist.

30. Zusammensetzung nach Anspruch 28 oder 29, **dadurch gekennzeichnet, dass** sie in Form einer Creme oder Lotion für die Haarbehandlung, eines Haarwaschmittels oder einer Haarspülung, einer Mascara für die Wimpern oder die Haare vorliegt.

31. Zusammensetzung nach einem der Ansprüche 28 bis 30, **dadurch gekennzeichnet, dass** sie in Form einer wässrigen, alkoholischen oder wässrig-alkoholischen Lösung oder Suspension vorliegt.

32. Zusammensetzung nach einem der Ansprüche 28 bis 31, **dadurch gekennzeichnet, dass** sie andere Bestandteile enthält, die unter den Lösemitteln, den Verdickungsmitteln oder Gelbildnern für die wässrige Phase oder die Ölphase, den in dem Medium der Zusammensetzung löslichen Farbstoffen, den Füllstoffen, den Pigmenten, den Antioxidantien, den Konservierungsmitteln, den Parfüms, den Elektrolyten, den Neutralisierungsmitteln, den filmbildenden Polymeren, den UV-Blockern, den kosmetischen und pharmazeutischen Wirkstoffen mit günstiger Wirkung für die Haut oder die Keratinfasern, deren Gemischen ausgewählt werden.

33. Zusammensetzung nach einem der Ansprüche 28 bis 32, **dadurch gekennzeichnet, dass** sie mindestens eine zusätzliche Verbindung enthält, die das Haarwachstum fördert und/oder den Haarausfall begrenzt, die ausgewählt wird unter Aminexil, 6-0-[(9Z,12Z)-Octadeca-9,12-dienoyl]-hexapyranose, den Inhibitoren der Lipoxygenase, den Inhibitoren von Bradykinin, den Prostaglandinen und deren Derivaten, den Agonisten oder Antagonisten der Rezeptoren der Prostaglandine, den nichtprostanischen Analoga der Prostaglandine, den gefäßerweiternden Mitteln, den antiandrogenen Wirkstoffen, den Cyclosporinen und deren Analoga, den antimikrobiellen Mitteln, den entzündungshemmenden Mitteln, den Retinoiden, Benzalkoniumchlorid, Benzethoniumchlorid, Phenol, Östradiol, Chlorpheniraminmaleat, den Chlorophyllin-Derivaten, Cholesterin, Cystein, Methionin, Menthol, Pfefferminzöl, Calciumpanthotenat, Panthenol, Resorcin, den Aktivatoren der Proteinkinase C, den Inhibitoren der Glycosidase, den Inhibitoren der Glycosaminoglycanase, den Estern der Pyroglutaminsäure, den Hexosaccharidsäuren oder Acylhexosaccharidsäuren, den substituierten Arylethylenen, den N-acylierten Aminosäuren, den Flavonoiden, den Derivaten und Analoga von Ascomycin, den Antagonisten von Histamin, den Saponinen, den Inhibitoren der Proteoglycanase, den Agonisten und Antagonisten der Östrogene, den Pseudoterinen, den Cytokinen und den Promotoren der Wachstumsfaktoren, den Inhibitoren von IL-1 oder IL-6, den Promotoren von IL-10, den Inhibitoren von TNF, den Vitaminen, den Hydroxysäuren, den Benzophenonen, Hydantoin, Octopirox, Retinoesäure, den Mitteln gegen Juckreiz, den antiparasitären Mitteln, den Antipilzmitteln, den Nicotinsäureestern, den Calciumantagonisten, den Hormonen, den Triterpenen, den antiandrogenen Mitteln, den steroidischen oder nichtsteroidischen Inhibitoren der 5-α-Reduktasen, den Agonisten der Kaliumkanäle, den Agonisten des FP-Rezeptors, den Inhibitoren von 15-Hydroxyprostaglandindehydrogenase, deren Gemischen.

34. Zusammensetzung nach einem der Ansprüche 28 bis 33, **dadurch gekennzeichnet, dass** sie außerdem einen zusätzlichen Wirstoff enthält, der unter den Proteinen, den Proteinhydrolysaten, den Aminosäuren, den Polyolen, Harnstoff, Allantoin, den Zuckern und Zuckerderivaten, den pflanzlichen Extrakten (die von Iridaceen oder Soja), Hydroxysäuren; den Derivaten von Retinol oder Tocopherol, den essentiellen Fettsäuren, den Ceramiden, den etherischen Ölen, den Salicylsäurederivaten, den Estern von Hydroxysäuren, den Phospholipiden, deren Gemischen ausgewählt wird.

35. Verfahren zur Herstellung eines Pyrimidin-*N*-oxid-Derivats nach einem der Ansprüche 1 bis 11, das die folgenden Schritte umfasst
1°) Umsetzung der Verbindung **5** 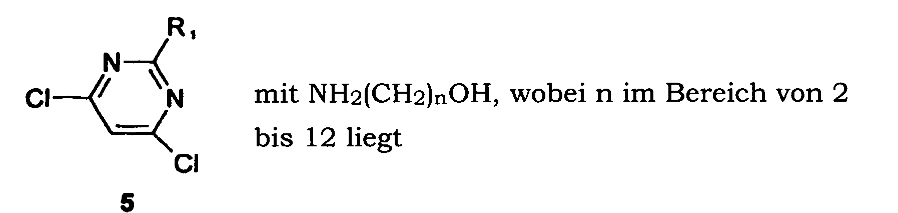
2°) Oxidation des in Schritt 1°) erhaltenen Produkts,
3°) Substitution des in Schritt 2°) erhaltenen Produkts, bei der ein Chloratom durch einen Substituenten ersetzt wird,
4°) Nitrierung des in Schritt 3°) erhaltenen Produkts.
